(19) Europäisches Patentamt
European Patent Office
Office européen des brevets

(11) Veröffentlichungsnummer: **0 387 582 B1**

(12) # EUROPÄISCHE PATENTSCHRIFT

(45) Veröffentlichungstag der Patentschrift: **27.07.94**

(51) Int. Cl.[5]: **C07D 471/04**, C07D 471/14, //(C07D471/04,221:00,221:00), (C07D471/14,231:00,221:00)

(21) Anmeldenummer: **90103685.5**

(22) Anmeldetag: **26.02.90**

(54) **2,3-substituierte 1,8-Naphthyridine, Verfahren zu ihrer Herstellung und ihre Verwendung als Antidots.**

(30) Priorität: **11.03.89 DE 3907938**

(43) Veröffentlichungstag der Anmeldung:
**19.09.90 Patentblatt 90/38**

(45) Bekanntmachung des Hinweises auf die Patenterteilung:
**27.07.94 Patentblatt 94/30**

(84) Benannte Vertragsstaaten:
**BE CH DE ES FR GB IT LI NL**

(56) Entgegenhaltungen:
**EP-A- 0 228 598** **EP-A- 0 230 235**
**EP-A- 0 230 239** **EP-A- 0 275 520**
**DE-A- 2 433 067** **DE-A- 3 004 770**

**JOURNAL OF MEDICINAL CHEMISTRY, Band 20, n0.1, 1977, Seiten 124-128; D.K.J. GOREKKI et al.: "2,3-Disubstituted 1,8-Naphthyridines as Potential Diuretic Agents" * Seite 124 „ Shema I; Seitennn 126, 127 , Tabelle I, Vergindungen Nr. 23,29,31-34, 50-52***

(73) Patentinhaber: **BASF Aktiengesellschaft**
**Carl-Bosch-Strasse 38**
**D-67063 Ludwigshafen(DE)**

(72) Erfinder: **Hagen, Helmut, Dr.**
**Max-Slevogt Strasse 17e**
**D-6710 Frankenthal(DE)**
Erfinder: **Pfister, Juergen, Dr.**
**Ziegelofenweg 1**
**D-6720 Speyer(DE)**
Erfinder: **Ziegler, Hans, Dr.**
**Pfalzring 91**
**D-6704 Mutterstadt(DE)**
Erfinder: **Wuerzer, Bruno, Dr.**
**Ruedigstrasse 13**
**D-6701 Otterstadt(DE)**
Erfinder: **Westphalen, Karl-Otto, Dr.**
**Mausbergweg 58**
**D-6720 Speyer(DE)**

**EP 0 387 582 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF MEDICINAL CHEMISTRY, Band 20, Nr.6, 1977, Seiten 838-841; E.M. HAWES et al.:"2,3-Disubstituted 1,8-Naphthyridines as Potential Diuretic Agents" *Seite 839, Tabelle I; Schema 1*

JOURNAL OF THE INDIAN CHEMICAL SOCIETY, Band 64, 1987, Seiten 193,194; K.R. Reddy et al.: "Substtuted 1,8-Naphtyridines. Part IV. Sythesis of 2-Methyl-N-aryl-1,8-naphthyridine-3-card oxamides"

TETRAHEDRON, Band 36, Nr. 17, 1980, Seiten 2359-2407; P. CALUWE et al.:" Heteroannaletations with o-Aminoaldehydes"* Seiten 2391-2394*

JOURNAL OF THE CHEMICAL SOCIETY , 1967, Seiten 1564-1568; E.M. HAWES et al.: + "1,8-Naphthyridines. Part II. Preparation and Some Reactions of 2-Substituted Derivatives"*Sete 1564, Formel (I)

## Beschreibung

Die vorliegende Erfindung betrifft 2,3-substituierte 1,8-Naphthyridine der allgemeinen Formel I

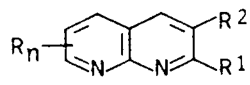

I,

in der die Substituenten die folgende Bedeutung haben:

R Wasserstoff oder $C_1$-$C_4$-Alkyl (n = 1 oder 2)

$R^1$ Amino oder $NR^3R^4$, wobei

$R^3$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxi, $C_1$-$C_4$-Halogenalkyl, Nitro, Amino, Mono- oder Dialkylamino mit 1 bis 6 C-Atomen je Alkylrest substituiertes Phenyl oder Phenyl-($C_1$-$C_3$)-alkyl oder eine Gruppe

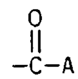

,

worin A für $C_1$-$C_8$-Alkyl, $C_2$-$C_6$-Alkenyl, Phenyl, ein- bis dreifach durch Halogen und/oder $C_1$-$C_4$-Alkyl substituiertes Phenyl, Amino, Alkyl- oder Dialkylamino mit jeweils 1 bis 6 C-Atomen je Alkylrest, Phenylamino, Morpholino oder Piperidyl steht, und

$R^4$ Wasserstoff, $C_1$-$C_{12}$-Alkyl, $C_3$-$C_8$-Cycloalkyl bedeuten oder

$R^3$ und $R^4$ miteinander zu einem gesättigten oder ungesättigten 5- oder 6-gliedrigen Heterocyclus, der neben dem Stickstoff, an den sie gebunden sind ein weiteres Heteroatom wie Sauerstoff oder Stickstoff enthalten kann, verbunden sind;

Hydrazino, ein- oder zweifach durch $C_1$-$C_4$-Alkyl oder einfach durch $C_1$-$C_4$-Acyl substituiertes Hydrazino;

$XR^5$, worin X Sauerstoff oder Schwefel bedeutet und $R^5$ für Wasserstoff, $C_1$-$C_{12}$-Alkyl, $C_1$-$C_4$-Alkoxicarbonyl-($C_1$-$C_4$)-alkyl, $C_5$-$C_8$-Cycloakyl, Phenyl, Phenyl-$C_1$-$C_3$-alkyl, Phenyl oder Phenyl-$C_1$-$C_3$-alkyl, jeweils am Ring substituiert durch Halogen, $C_1$-$C_4$-Alkyl, $C_1$-$C_4$-Alkoxi, Amino, Mono- oder Dialkylamino mit 1 bis 6 C-Atomen je Alkylrest steht,

Halogen,

Isothiorhoniumhalogenid,

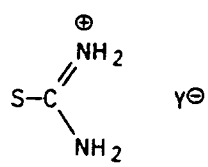

,

wobei Y für Fluor, Chlor, Brom oder Iod steht,

$C_1$-$C_{12}$-Alkyl,

$C_1$-$C_4$-Alkoxycarbonyl-$C_1$-$C_4$-alkyl, Phenyl oder Phenyl-($C_1$-$C_3$)-alkyl, wobei die aromatischen Reste ein bis drei der folgenden Gruppen tragen können: Halogen, $C_1$-$C_4$-Alkyl, $C_1$-$C_4$-Alkoxy, Amino, Nitro, Mono- oder Dialkylamino mit jeweils 1 bis 4 C-Atomen im Alkylrest,

$R^2$ $C_1$-$C_4$-Alkyl, Cyano,

Carboxyl,

eine Gruppe

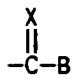

oder

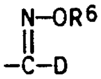

,

worin

- X Sauerstoff oder Schwefel,
- B $C_1$-$C_6$-Alkyl, $C_1$-$C_6$-Alkoxy oder Alkylthio, Amino, Mono- oder Dialkylamino mit 1 bis 4 C-Atomen je Alkylrest, Morpholino, Piperidyl, Chlor, Brom oder Phenyl, welches seinerseits ein bis fünf Halogenatome und/oder ein bis drei der folgenden Gruppen tragen kann: $C_1$-$C_4$-Alkyl, $C_1$-$C_4$-Alkoxy, $C_1$-$C_4$-Alkoxy, $C_1$-$C_4$-Halogenalkyl, $C_1$-$C_4$-Halogenalkoxy und/oder $C_1$-$C_4$-Alkylthio;
- D $C_1$-$C_4$-Alkyl oder $NH_2$ und
- $R^6$ Wasserstoff, $C_1$-$C_8$-Alkyl oder $C_2$-$C_8$-Alkylcarbonyl bedeuten oder $R^1$ und $R^2$ gemeinsam $-NH-N=C(NH_2)-$

sowie deren pflanzenverträgliche Salze

ausgenommen 2-Chlor-1,8-naphthyridin-3-carbonitril, 2-Methoxy-1,8-naphthyridin-3-carbonitril, 2-Methylamino-1,8-naphthyridin-3-carbonitril,2-Dimethylamino-1,8-naphthyridin-3-carbonitril,2-Diacetylamino-1,8-naphthyridin-3-carbonitril, 2-Phenyl-1,8-naphthyridin-3-carbonitril, 5,7-Dimethyl-2-phenyl-1,8-naphthyridin-3-carbonitril, 2,3,5,7-Tetramethyl-1,8-naphthyridin,2-Methylamino-1,8-naphthyridin-3-carboxamid, 2-Dimethylamino-1,8-naphthyridin-3-carboxamid, 2-Acetyl-amino-1,8-naphthyridin-3-carboxamid, 2-Phenyl-1,8-naphthyridin-3-carboxamid und 2-Amino-1,8-naphthyridin-3-thio-carboxamid,

sowie diejenigen Verbindungen I, bei denen $R^1$ Methyl, Hydroxyl oder Amino ($NH_2$) bedeutet und gleichzeitig $R^2$ für Cyano, Carboxyl, eine Carbonsäureamid- oder Carbonsäureestergruppe steht.

Außerdem betrifft die Erfindung Verfahren zur Herstellung der Verbindungen I sowie herbizide Mittel, die 2-(4-Heteroaryloxy)- oder 2-(4-Aryloxy)-phenoxyessig- oder -propionsäurederivate und/oder Cyclohexenonderivate als herbizide Wirkstoffe und 2,3-substituierte 1,8-Naphthyridine als Antidots enthalten, sowie Verfahren zur selektiven Bekämpfung von unerwünschtem Pflanzenwuchs mit diesen herbiziden Mitteln.

Von P. Caluwe wird die Herstellung von verschiedenen 1,8-Naphthyiridin-derivaten in Tetrahedron, Bd. 36, Seiten 2359 bis 2407, insbesondere S. 2391 bis 2394, 1980 beschrieben. 2-Amino-1,8-naphthyiridine mit Carbonsäure(derivat)resten in 3-Stellung sind aus J. Chem. Soc. (C), 1564 bis 1568, 1967 bekannt. Eine pflanzenschützende Wirkung dieser Verbindungsklasse ist dem Stand der Technik nicht zu entnehmen.

Herbizide Wirkstoffe aus der Gruppe der 2-(4-Heteroaryloxy)- oder 2- (4-Aryloxy)-phenoxyessigsäurederivate der Formel IV,

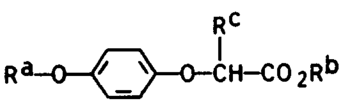

IV

in der die Substituenten folgende Bedeutung haben:

- $R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, $C_1$-$C_4$-Alkyl, $C_1$-$C_4$-Halogenalkyl und/oder Nitro;
- $R^b$ Wasserstoff, eine $C_1$- $C_4$-Alkylgruppe oder das Äquivalent eines pflanzenverträglichen Kations und
- $R^c$ Wasserstoff oder eine Methylgruppe,

sind aus der Literatur, z.B. aus DE-A-22 23 894, DE-A-24 33 067, DE-A-25 76 251, DE-A-30 04 770, BE-A-868 875 und BE-A-858 618 bekannt.

Sie dienen der Bekämpfung von unerwünschten Pflanzen aus der Familie der Gramineen. Die Verträglichkeit dieser Substanzen für Kulturpflanzen variiert jedoch zwischen kommerziell akzeptabel und unverträglich, je nach Substituenten und Aufwandmenge.

Ebenso verhält es sich mit Cyclohexenonderivaten der Formel V,

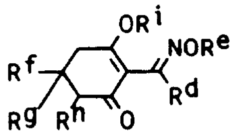

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann;

ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;

einen 10-gliedrigen gesättigter oder einfach ungesättigter Heterocyclus, welcher zwei Sauerstoffatome oder Schwefelatome enthält und durch bis zu drei $C_1$-$C_4$-Alkylgruppen und/oder Methoxygruppen substituiert sein kann;

eine Phenylgruppe, eine Pyridylgruppe, eine Thiazolylgruppe, eine Pyrazolylgruppe, eine Pyrrolylgruppe oder eine Isoxazolylgruppe, wobei diese Gruppen bis zu drei der folgenden Reste tragen können:

$C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom oder eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

Sie sind in der Literatur (z.B. EP-A 228 598, EP-A 230 235, EP-A 238 021, US-A 4 432 786, DE-A 24 39 104) ebenfalls als Herbizide beschrieben und dienen vorwiegend zur Bekämpfung unerwünschter Gräser in dicotylen Kulturen und in Kulturpflanzen die zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Der Erfindung lag nun die Aufgabe zugrunde, Verbindungen zu finden, welche die Nachteile, die bei der Verwendung der obengenannten Herbizide der Formeln IV und V bestehen, zumindest so stark zu verringern, daß die Herbizide für die Kulturpflanzen aus der Familie der Gräser (Gerste, Weizen, Mais, Kultursorghum und Reis) verträglich werden.

Entsprechend der Aufgabe wurden die eingangs definierten 2,3-substituierten 1,8-Naphthyridine I gefunden. Weiterhin wurden Verfahren zur Herstellung dieser Verbindungen I sowie zur gemeinsamen Anwendung dieser Verbindungen mit den Herbiziden IV und V zur Beeinflussung unerwünschten Pflanzenwachstums gefunden. Weiterhin betrifft die Erfindung Mittel, welche die Verbindungen I sowie die durch den Disclaimer ausgeschlossenen Verbindungen enthalten, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Die erfindungsgemäßen Verbindungen I sind ausgehend von o-Aminopicolinaldehyd II und Umsetzung mit einer CH-aciden Verbindung IIIa oder IIIb zugänglich.

Das Substitutionsmuster kann durch entsprechende Wahl der CH-aciden Verbindung III in einem weiten Bereich variiert werden; neben Acetyl- und 1,3-Dicarbonylverbindungen können auch $\beta$-Ketosäure-, Acetondicarbonsäure-, Malonsäure- und Cyanessigsäureester sowie Malodinitril für die Cyclodehydratisierung eingesetzt werden.

Die Herstellung von I sei anhand folgender Reaktionsgleichungen demonstriert:

a) $R^1 \neq NH_2$, OH

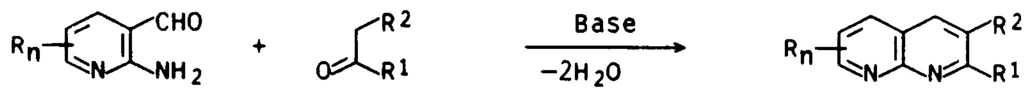


IIIa I

z.B. mit

$R^1$ = OAlkyl, OAryl, $CH_2COOAlkyl$, COOAlkyl und

$R^2$ = H, Alkyl, Alkylcarbonyl, COOAlkyl und

R = H oder Methyl

b) $R^1$ = $NH_2$, OH

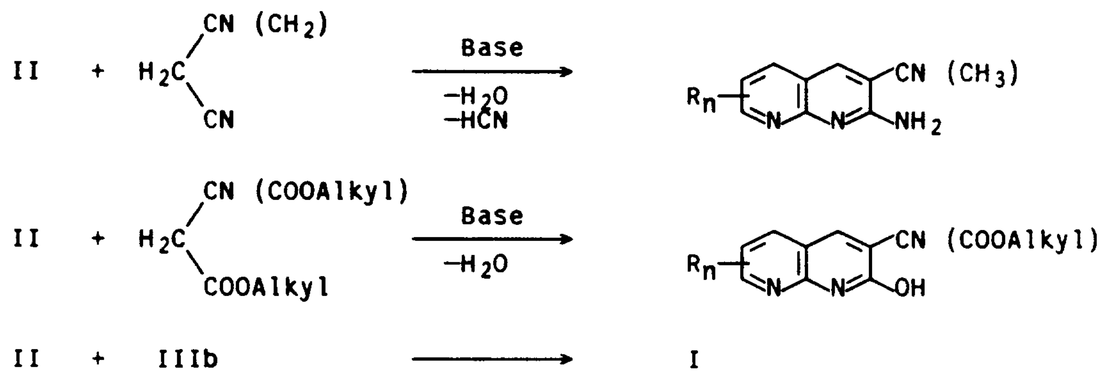


2-Hydroxynaphthyridine reagieren mit einem Halogenierungsmittel wie Phosphoroxychlorid oder -bromid zu 2-Halogennaphthyridinen. Durch Austauschreaktionen mit N- oder S-Nukleophilen kann das Substitutionsmuster in einem weiten Bereich variiert werden. Neben primären und sekundären Aminen eignen sichhierfür auch Mercaptane, Thioharnstoffe und Rhodanide (vgl. z.B. The Chemistry of Heterocyclic Compounds Band 14, Teil 1-5, E. Klingsberg, Ed. Interscience, New York/London, 1960 und Comprehensive Heterocyclic Chemistry A.R. Hatritiky, Ed. Pergamon Press 1984, Teil 2, S. 359-364.

Üblicherweise setzt man die Ausgangsstoffe II und III in stöchiometrischem Verhältnis ein. Ein Überschuß des einen oder anderen kann in Einzelfällen aber durchaus vorteilhaft sein.

Die Umsetzung kann kontinuierlich oder diskontinuierlich bei Normal-, über- oder Unterdruck nach den dafür üblichen Techniken durchgeführt werden. Die Reaktionstemperatur liegt im allgemeinen bei 20 bis 200, insbesondere bei 50 bis 150 °C, vorteilhaft im Siedebereich des Lösungsmittels.

Als Lösungsmittel dienen beispielsweise aliphatische und aromatische, gegebenenfalls chlorierte Kohlenwasserstoffe wie Petrolether, Benzol, Toluol, Xylol, Benzin, Dichlormethan, Chloroform, Tetrachlormethan, 1,2-Dichlorethan, Chlorbenzol, höhersiedende Ether wie Tetrahydrofuran oder Dioxan; Nitrile wie Acetonitril und Propionitril.

Als Basen dienen insbesondere aliphatische aromatische oder heterocyclische Amine, beispielsweise Triethylamin, Dimethylamin, Diisopropylamin, Piperidin, Dimethylanilin, Dimethylbenzylamin, Pyridin und 4-Dimethylaminopyridin. Hydroxide von Alkali- und Erdalkalimetallen wie z.B. Natriumhydroxid, Kaliumhydroxid, Calciumhydroxid; Alkoholate von Alkali- und Erdalkalimetallen wie beispielsweise Natriummethylat, Natriumethylat, Calciummethylat, Kalium-tert.-butylat; Alkali- oder Erdalkalimetallhydride wie beispielsweise Natriumhydrid, Kaliumhydrid oder Calciumhydrid.

Unter Umständen kann es vorteilhaft sein, die Kondensation in Gegenwart eines üblichen Phasentransferkatalysators vorzunehmen.

Im Hinblick auf die bestimmungsgemäße Verwendung der Verbindungen I als pflanzenschützende Mittel kommen als Substituenten folgende Reste in Betracht:

| | |
|---|---|
| R | Wasserstoff oder $C_1$-$C_4$-Alkyl wie Methyl, Ethyl, Propyl, iso-Propyl, Butyl, iso-Butyl, bevorzugt in 5- und/oder 7-Position, |
| n | eine Zahl 1 oder 2, |
| $R^1$ | Amino oder $NR^3R^4$, wobei |
| $R^3$ | $C_1$-$C_{12}$-Alkyl, insbesondere $C_1$-$C_8$-, bevorzugt $C_1$-$C_4$-Alkyl wie Methyl, Ethyl, Propyl, iso- |

Propyl, n-Butyl, sec-Butyl, iso-Butyl und tert.-Butyl,
$C_3$-$C_6$-Alkenyl wie z.B. Allyl, 2-Methylallyl oder But-3-enyl,
$C_3$-$C_8$-Cycloalkyl, insbesondere Cyclopentyl und Cyclohexyl,
Phenyl, ggf. substituiert durch Halogen, $C_1$-$C_4$-Alkyl, $C_1$-$C_4$-Alkoxi, $C_1$-$C_4$-Halogenalkyl, wie Trifluormethyl, Nitro, Amino, Mono- oder Dialkylamino wie Methylamino, Dimethylamino, Ethylamino, Diethylamino, Propylamino, Isopropylamino oder tert. -Butylamino,
Phenyl-($C_1$-$C_3$)-alkyl wie Benzyl, Phenylethyl, wobei der Phenylkern wie o.g. substituiert sein kann,
eine Gruppe

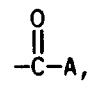

worin A für $C_2$-$C_6$-Alkenyl, insbesondere $C_2$-$C_4$-Alkenyl wie Ethenyl, Prop-3-enyl und But-3-enyl, $C_1$-$C_8$-Alkyl, insbesondere $C_1$-$C_4$-Alkyl wie für $R^3$ genannt, Phenyl, das unsubstituiert oder durch $C_1$-$C_4$-Alkyl oder Halogen wie Fluor, Chlor oder Brom substituiert ist, Amino, Alkyl- oder Dialkylamino mit jeweils 1 bis 6 C-Atomen, insbesondere 1 bis 4 C-Atomen je Alkylrest wie z.B. Methylamino, Dimethylamino, Ethylamino, Diethylamino, Propylamino, Dipropylamino, Diisopropylamino oder tert.-Butylamino oder Di-n-butylamino, Phenylamino, Morpholino oder Piperidyl,

$R^4$ Wasserstoff,
$C_1$-$C_{12}$-Alkyl, insbesondere $C_1$-$C_8$-, bevorzugt $C_1$-$C_4$-Alkyl wie für $R^3$ genannt oder $C_3$-$C_8$-Cycloalkyl, z.B. Cyclopentyl oder Cyclohexyl oder

$R^3$ und $R^4$ miteinander zu einem gesättigten oder ungesättigten 5- oder 6-gliedrigen Heterocyclus, der neben dem Stickstoff, an den sie gebunden sind, ein weiteres Heteroatom wie Sauerstoff oder Stickstoff enthalten kann, verbunden sind, z.B. zu einem Piperidyl-, Piperazinyl, Morpholinyl, Pyrrolidinyl, Azolyl- oder Imidazolylring.
Hydrazino, das ggf. substituiert ist durch ein bis drei $C_1$-$C_4$-Alkylreste, insbesondere durch zwei geminal oder vicinal angeordnete $C_1$-$C_4$-Alkylreste wie z.B. Methyl oder Ethyl oder monoacyliert ist, wobei als Acylrest $C_1$-$C_4$-Acyl, insbesondere Acetyl in Betracht kommt,
$XR^5$, worin X Sauerstoff oder Schwefel bedeutet und $R^5$ für Wasserstoff, $C_1$-$C_{12}$-Alkyl wie für $R^3$ genannt, $C_1$-$C_4$-Alkoxicarbonyl-($C_1$-$C_4$)-alkyl wie $CH_2COOCH_3$ oder $CH_2COOC_2H_5$, $C_5$-$C_8$-Cycloalkyl wie für $R^3$ genannt und ggf. substituiertes Phenyl oder Phenyl-($C_1$-$C_3$)-alkyl wie unter $R^3$ aufgeführt steht,
Halogen,
Isothiorhoniumhalogenid

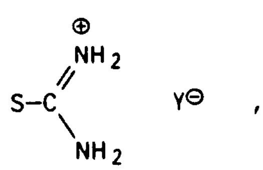

wobei Y für Fluor, Chlor, Brom oder Iod, insbesondere Chlor oder Brom steht,
$C_1$-$C_{12}$-Alkyl wie für $R^3$ genannt, $C_1$-$C_4$-Alkoxycarbonyl-$C_1$-$C_4$-alkyl, insbesondere $C_1$-$C_3$-Alkoxycarbonyl-$C_1$-$C_2$-alkyl, wie Methoxycarbonylmethyl, Ethoxycarbonylmethyl, 1-(Methoxycarbonyl)ethyl und 1-(Ethoxycarbonyl)ethyl,
Phenyl oder Phenyl-($C_1$-$C_3$)-alkyl wie Benzyl oder Phenylethyl, wobei der Phenylkern jeweils durch Halogen, $C_1$-$C_4$-Alkyl, $C_1$-$C_4$-Alkoxi, $NH_2$, $NO_2$, Mono- oder Dialkylamino mit jeweils 1 bis 4 C-Atomen je Alkylrest ein- bis dreifach substituiert sein kann,

$R^2$ $C_1$-$C_4$-Alkyl wie Methyl, Ethyl, Propyl, 1-Methylethyl, Butyl, 1-Methylpropyl, 2-Methylpropyl und 1,1-Dimethylethyl, Cyano,
COOH,
eine Gruppe

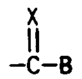 oder 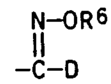 ,

worin
X Sauerstoff oder Schwefel bedeutet,
B für $C_1$-$C_6$-Alkoxy oder -Alkylthio wie Methoxy, Ethoxy, Propoxy, i-Propoxy, Butoxy, Methylthio, Ethylthio, Propylthio oder Butylthio, Phenyl, Amino, $C_1$-$C_4$-Monoalkylamino wie z.B. Methylamino, Ethylamino, Propylamino oder $C_2$-$C_8$-Dialkylamino wie z.B. Dimethylamino, Diethylamino, Dipropylamino oder Dibutylamino, Morpholino, Piperidyl, $C_1$-$C_6$-Alkyl wie für $R^3$ genannt, sowie n-Pentyl, 2-Methylbutyl, 3-Methylbutyl, 2,2-Dimethylpropyl, n-Hexyl, 2-Methylpentyl, 3-Methylpentyl, 4-Methylpentyl, 2,2-Dimethylbutyl, 3,3-Dimethylbutyl, 2,3-Dimethylbutyl oder 2-Ethylbutyl, Chlor oder Brom oder Phenyl, welches seinerseits ein bis drei der folgenden Gruppen tragen kann: Halogen wie insbesondere Fluor, Chlor und Brom, Alkyl jwie insbesondere Methyl, Ethyl und iso-Propyl, Alkoxy wie insbesondere Methoxy, Halogenalkyl wie insbesondere Difluormethyl, Trifluormethyl und Chlormethyl, Halogenalkoxy wie insbesondere Difluormethoxy und Trifluormethoxy und/oder Alkylthio wie insbesondere Methylthio und Ethylthio,
D für $C_1$-$C_4$-Alkyl wie für $R^3$ genannt oder $NH_2$ und $R^6$ für Wasserstoff, $C_1$-$C_8$-Alkyl wie für $R^3$ genannt oder $C_1$-$C_8$-Alkylcarbonyl wie Acetyl, Ethylcarbonyl, Propylcarbonyl, 1-Methylethylcarbonyl, Butylcarbonyl, 1-Methylpropylcarbonyl, 2-Methylpropylcarbonyl oder 1, 1-Dimethylethylcarbonyl,insbesondere Acetyl oder $R^1$ und $R^2$ gemeinsam -NH-H=C-($NH_2$)- stehen

und deren pflanzenverträgliche Salze.

Spezielle Beispiele für herbizide (Heteroaryloxy)- bzw. Aryloxy-phenoxyessigsäurederivate der Formel IV, deren Kulturpflanzenverträglichkeit durch 2,3-substituierte 1,8-Naphthyridine der Formel I verbessert werden kann, sind in der folgenden Tabelle A aufgeführt:

Tabelle A

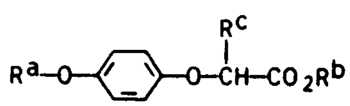 IV

| Nr. | $R^a$ | $R^b$ | $R^c$ | Lit |
|---|---|---|---|---|
| IV.1 | 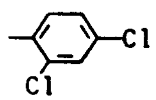 | $CH_3$ | $CH_3$ | DE-A 22 23 894 |
| IV.2 | 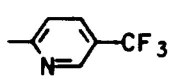 | n-$C_4H_9$ | $CH_3$ | BE-A 868 875 |
| IV.3 | 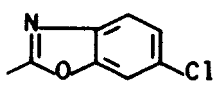 | $C_2H_5$ | $CH_3$ | BE-A 858 618 |
| IV.4 | 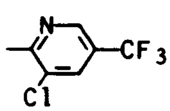 | $CH_3$ | $CH_3$ | BE-A 868 875 |
| IV.5 | 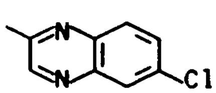 | $C_2H_5$ | $CH_3$ | DE-A 30 04 770 |

Spezielle Beispiele für Cyclohexenone der Formel V, deren Kulturpflanzenverträglichkeit durch 2,3-substituierte 1,8-Naphthyridine der Formel I verbessert werden kann, sind in der folgenden Tabelle B aufgeführt.

Tabelle B

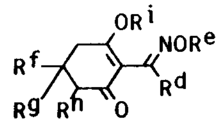

V

| Nr. | $R^d$ | $R^e$ | $R^f$ | $R^g$ | $R^h$ | $R^i$ | Literatur |
|---|---|---|---|---|---|---|---|
| V.1 | $C_3H_7$ | $CH_2CH=CH_2$ | $CH_3$ | $CH_3$ | $CO_2CH_3$ | Na | DE-A 2 439 104 |
| V.2 | $C_3H_7$ | $CH_2CH_3$ | $CH_2CH(CH_3)SCH_2CH_3$ | H | H | H | DE-A 2 822 304 |
| V.3 | $C_2H_5$ | $CH_2CH=CHCl$ | $CH_2CH(CH_3)SCH_2CH_3$ | H | H | H | US-A 4 440 566 |
| V.4 | $C_3H_7$ | $CH_2CH=CHCl$ | $CH_2CH(CH_3)SCH_2CH_3$ | H | H | H | US-A 4 440 566 |
| V.5 | $C_3H_7$ | $C_2H_5$ | 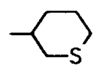 | H | H | H | EP-A 71 707 |
| V.6 | $C_2H_5$ | $C_2H_5$ | 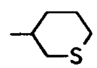 | H | H | H | EP-A 71 707 |
| V.7 | $CH_3$ | $CH_2CH=CHCH_3$ | 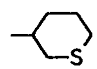 | H | H | H | EP-A 71 707 |
| V.8 | $C_3H_7$ | $C_2H_5$ | 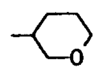 | H | H | H | EP-A 71 707 |
| V.9 | $C_2H_5$ | $CH_2CH=CHCl$ | 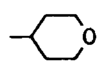 | H | H | H | EP-A 142 741 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.10 | $C_3H_7$ | $C_2H_5$ | 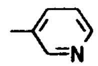 | H | H | H | EP-A 66 195 |
| V.11 | $C_2H_5$ | $C_2H_5$ | 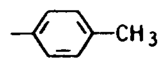 | H | H | H | DE-A 24 39 104 |
| V.12 | $C_2H_5$ | $CH_2CH=CHCH_3$ | 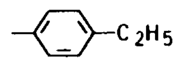 | H | H | H | DE-A 38 08 072 |
| V.13 | $C_2H_5$ | $C_2H_5$ | 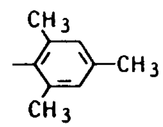 | H | H | H | EP-A 88 301 |
| V.14 | $C_3H_7$ | $CH_2CH=CHCl$ | 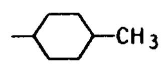 | H | H | H | EP-A 88 299 |
| V.15 | $C_3H_7$ | $CH_2CH=CHCH_3$ | 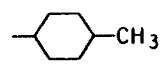 | H | H | H | EP-A 88 299 |
| V.16 | $C_2H_5$ | $CH_2CH=CHCH_3$ | 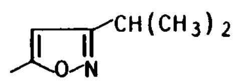 | H | H | H | EP-A 238 021 |
| V.17 | $C_3H_7$ | $CH_2CH=CHCH_3$ | 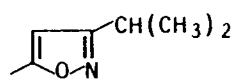 | H | H | H | EP-A 238 021 |
| V.18 | $C_2H_5$ | $CH_2CH=CHCl$ | 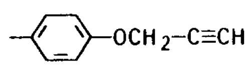 | H | H | H | EP-A 137 174 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.19 | $C_3H_7$ | $C_2H_5$ | 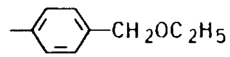 | H | H | H | EP-A 137 200 |
| V.20 | $C_3H_7$ | $C_2H_5$ | 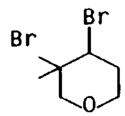 | H | H | H | EP-A 230 235 |
| V.21 | $C_3H_7$ | $CH_2CH=CHCl$ | 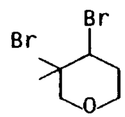 | H | H | H | EP-A 230 235 |
| V.22 | $C_3H_7$ | $CH_2CH=CHCl$ | 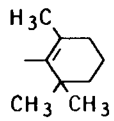 | H | H | H | EP-A 46 860 |
| V.23 | $C_3H_7$ | $C_2H_5$ | 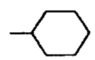 | H | H | H | JP-A 540 191 945 |
| V.24 | $C_3H_7$ | $C_2H_5$ | 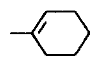 | H | H | H | EP-A 46 860 |
| V.25 | $CH_3$ | $CH_2CH=CHCl$ | 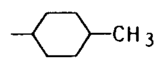 | H | H | H | EP-A 88 299 |
| V.26 | $C_3H_7$ | $C_2H_5$ | 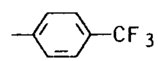 | H | H | K | EP-A 137 174 |
| V.27 | $C_2H_5$ | $CH_2CH=CHCl$ | 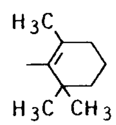 | H | H | H | EP-A 46 860 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.28 | $C_3H_7$ | $CH_2CH{=}CHCH_3$ | 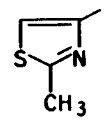 | H | H | H | EP-A-125 094 |
| V.29 | $C_3H_7$ | $CH_2CH{=}CHCl$ | 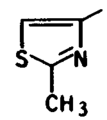 | H | H | H | EP-A-125 094 |
| V.30 | $C_3H_7$ | $C_2H_5$ | 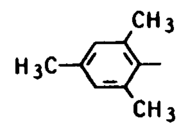 | H | H | H | EP-A-88 299 |
| V.31 | $C_3H_7$ | $CH_2CH{=}CH_2$ | 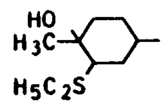 | H | H | H | EP-A-228 598 |
| V.32 | $C_2H_5$ | $C_2H_5$ | 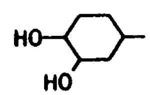 | H | H | H | EP-A-228 598 |
| V.33 | $C_3H_7$ | $C_2H_5$ | 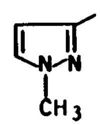 | H | H | H | EP-A-66 195 |

Tabelle B (Fortsetzung)

| Nr. | $R^d$ | $R^e$ | $R^f$ | $R^g$ | $R^h$ | $R^i$ | Literatur |
|---|---|---|---|---|---|---|---|
| V.34 | $C_3H_7$ | $CH_2CH{=}CHCl$ | 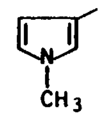 | H | H | H | EP-A-66 195 |
| V.35 | $C_3H_7$ | $CH_2CH{=}CH_2$ | 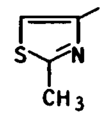 | H | H | H | EP-A-125 094 |
| V.36 | $C_3H_7$ | $C_3H_7$ | $CH(SCH_2CH_3)_2$ | H | H | H | EP-A-230 260 |
| V.37 | $C_3H_7$ | $C_2H_5$ | 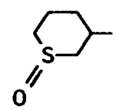 | H | H | H | EP-A-115 808 |
| V.38 | $C_3H_7$ | $C_2H_5$ | 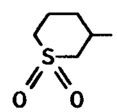 | H | H | H | EP-A-115 808 |
| V.39 | $C_3H_7$ | $C_3H_7$ | $CH_3$ | $CH_3$ | $C(CH_3){=}NOCH_3$ | H | EP-A-172 551 |
| V.40 | $C_3H_7$ | $CH_2CH{=}CH_2$ | 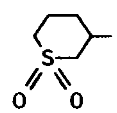 | OH | H | H | Proceedings Brit. Crop-Protection Conference-weeds 1985, Vol.1, S. 93-98 |

Zur selektiven Bekämpfung von unerwünschtem Pflanzenwuchs wird ein 2,3-disubstituiertes 1,8-Naphthyridin I und entweder ein 2-(4-Heteroaryloxy)- oder 2-(4-Aryloxy)phenoxyessigsäurederivat IV oder ein Cyclohexenonderivat V bei oder nach der Aussaat der Kulturpflanzen vor oder während dem Auflaufen der Kulturpflanzen gleichzeitig oder nacheinander ausgebracht. Nach dem Auflaufen können die herbiziden Wirkstoffe IV und V und die antidotisch wirkenden Verbindungen I gemeinsam oder getrennt auf die Blätter

und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Auch eine Behandlung der Kulturpflanzensamen mit dem Antidot vor der Aussaat reduziert oder vermindert die üblicherweise durch das verwendete Herbizid hervorgerufenen Schäden. Der herbizide Wirkstoff wird dann allein in der üblichen Weise appliziert.

Für herbizide (Heteroaryloxy)phenoxysäurederivate werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der (Heteroaryloxy)phenoxysäurederivate und der jeweiligen Zielkultur. Geeignete Anteilverhältnisse herbizider Wirkstoffe: antidotisch wirkender Verbindung liegen zwischen 1 : 4 bis 1 : 0,01; vorzugsweise zwischen 1 : 4 bis 1 : 0,1 Gew.-Teile.

Für das gleiche Cyclohexenonderivat werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein 1,8-Naphthyridinderivat I eingesetzt werden, sind in breiten Bereichen variabel. Sie sind abhängig von der Struktur des Cyclohexenonderivats, des Naphthyridinderivates I und der jeweiligen Kultur. Geeignete Anteilverhältnisse herbizider Wirkstoff: antidotisch wirkender Verbindung liegen zwischen 1 : 4 bis 1 : 0,01; vorzugsweise 1 : 4 bis 1 : 0,25 Gew.-Teile.

Die neuen herbiziden Mittel können neben dem Naphthyridinderivat I als Antidot und dem Herbizid aus der Gruppe der (Heteroaryloxy)phenoxyessigsäuren IV bzw. der Cyclohexenone V weitere herbizide oder wachstumsregulierende Wirkstoffe anderer chemischer Struktur enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), öldispersionen durch Zusatz von Wasser bereitet werden. Zur Herstellung von Emulsionen, Pasten oder öldispersionen können herbizider Wirkstoff und/oder Antidot als solche oder in einem öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton,

Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0, 1 bis 2 kg/ha.

Synthesebeispiele

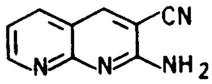

24,4 g (0,20 mol) 2-Aminonicotinaldehyd, 13,2 g (0,20 mol) Malonsäuredinitril und 2 ml Piperidin wurden in 120 ml Ethanol 15 h unter Rückfluß erhitzt. Nach dem Abkühlen saugte man ab und wusch den Niederschlag mit Ethanol nach.

Ausbeute: 23,0 g (85 % der Theorie) 2-Amino-1,8-naphthyridin-3-carbonitril mit Fp. 260 °C.

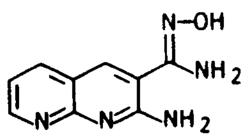

23,7 g (0,15 mol) 2-Amino-1,8-naphthyridin-3-carbonitril, 14,0 g (0,20 mol) Hydroxylammoniumchlorid und 16,8 g (0,20 mol) Natriumhydrogencarbonat wurden 15 h in 250 ml Ethanol/Wasser (3:2) unter Rückfluß erhitzt. Nach dem Abkühlen saugte man den Niederschlag ab und wusch mit n-Propanol nach.

Ausbeute: 22,3 g (69 %) 2-Amino-1,8-naphthyridin-3-carbonsäureamidoxim mit Fp. 280 °C.

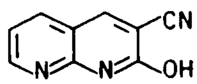

Eine Mischung aus 12,2 g (0,10 mol) 2-Aminonicotinaldehyd, 12,8 g (0,12 mol) Cyanessigsäureethylester, 1 ml Pyridin und 200 ml Ethanol wurde 30 min unter Rückfluß zum Sieden erhitzt. Nach dem Abkühlen bildete sich ein Niederschlag, der in üblicher Weise isoliert und gereinigt wurde.

Ausbeute: 13,6 g 805 % der Theorie) 3-Cyano-2-hydroxy-1,8-naphthyridin mit Fp. >240 °C.

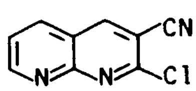

Eine Mischung aus 17,1 g (0,1 mol) 3-Cyano-2-hydroxy-1,8-naphthyridin und 50 ml Phosphoroxychlorid wurde 3 Stunden auf 100 °C erhitzt. Nach dem Abkühlen wurde die Reaktionsmischung in Wasser eingerührt. Durch alkalisch stellen der wäßrigen Mischung erhielt man einen Niederschlag, der in üblicher Weise isoliert und gereinigt wurde.

Ausbeute: 15,5 g (82 %) 2-Chlor-3-cyano-1,8-naphthyridin mit Fp. >240 °C.

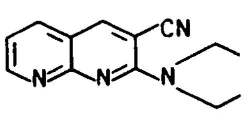

Eine Mischung aus 11,4 g (0,06 mol) 2-Chlor-2-cyano-1,8-naphthyridin, 15,4 ml (0,15 mol) Diethylamin und 100 ml Ethanol wurde 6 Stunden unter Rückfluß zum Sieden erhitzt. Die Reaktionsmischung wurde heiß filtriert und die so erhaltene Lösung wurde mit Wasser versetzt. Nach dem Abkühlen bildete sich ein Niederschlag, der in üblicher Weise isoliert und gereinigt wurde.

Ausbeute: 10,3 g (76 % der Theorie) 3-cyano-2-(N,N-diethylamino)-1,8-naphthyridin mit Fp. 119-121 ° C.

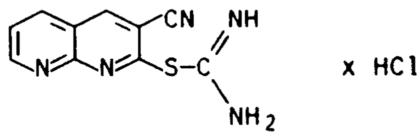

Eine Mischung aus 19 g (0,1 mol) 2-Chlor-3-cyano-1,8-naphthyridin, 7,6 g (0,1 mol) Thioharnstoff und 200 ml Ethanol wurde 1 Stunde auf 60 ° C erhitzt. Nach dem Abkühlen bildete sich ein Niederschlag, der in üblicher Weise isoliert und gereinigt wurde.

Ausbeute: 25; 1 g (94 % der Theorie) 3-Cyano-2-isothiuronio-1,8-naphthyridin-Hydrochlorid mit Fp. >240 ° C.

Tabelle 1: 2,3-substituierte 1,8-Naphthyridine

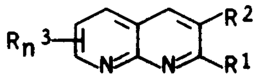

(I)

| Beispiel Nr. | $R^1$ | $R^2$ | $R_n^3$ | physik. Daten Fp (°C) bzw. Lit. [a]) |
|---|---|---|---|---|
| 1 | $NH_2$ | CN | H | (1), (2) |
| 2 | $NHC_2H_5$ | CN | H | |
| 3 | $NHCH_3$ | CN | H | |
| 4 | $N(C_2H_5)_2$ | CN | H | 119-121 |
| 5 | $NHCOC_2H_5$ | CN | H | 221-223 |
| 6 | $NHCH_2CH=CH_2$ | CN | H | 182-184 |
| 7 | $NHCOCH_3$ | CN | H | 200-205 |
| 8 | $NHCONHCH_3$ | CN | H | >240 |
| 9 | $NHCONH-n-C_4H_9$ | CN | H | 156-160 |
| 10 | $NHCONHC_6H_5$ | CN | H | 195 |
| 11 | Morpholino | CN | H | 194-196 |
| 12 | OH | CN | H | (2) |
| 13 | $S-C=NH_2^{\oplus}Cl^{\ominus}$<br>\|<br>$NH_2$ | CN | H | >240 |
| 14 | Cl | CN | H | >240 |
| 15 | $NH_2$ | COOH | H | (1) |
| 16 | OH | COOH | H | (2) |
| 17 | $CH_2COCH_3$<br>‖<br>O | $COOCH_3$ | H | 126-130 |
| 18 | OH | $COOC_2H_5$ | H | 203-205 |
| 19 | $N(C_2H_5)_2$ | $COOC_2H_5$ | H | |
| 20 | $S-C_{12}H_{25}$ | $COOC_2H_5$ | H | Oel |
| 21 | $S-C(NH_2)=NH_2^{\oplus}Cl^{\ominus}$ | $COOC_2H_5$ | H | 189 (Zers.) |
| 22 | Cl | $COOC_2H_5$ | H | 127-129 |
| 23 | $CH_3$ | $COOC_2H_5$ | H | 86- 88 |
| 24 | $NH_2$ | $CONH_2$ | H | (2) |
| 25 | $CH_3$ | CO- 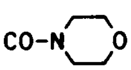 | H | 155-158 |

Tabelle 1 (Fortsetzung)

| Beispiel Nr. | $R^1$ | $R^2$ | $R_n^3$ | physik. Daten Fp (°C) bzw. Lit. a) |
|---|---|---|---|---|
| 26 | $NH_2$ | C=N-OH<br>\|<br>$NH_2$ | H | 280 (Zers.) |
| 27 | $NH_2$ | $CS-NH_2$ | H | |
| 28 | $CH_3$ | $COCH_3$ | H | 129-133 |
| 29 | $CH_3$ | C=N-OH<br>\|<br>$CH_3$ | H | 176-178 |
| 30 | $CH_3$ | $C=N-OCCH_3$ (C=O)<br>\|<br>$CH_3$ | H | 155-159 |
| 31 | $CH_3$ | $CH_3$ | H | 132-133 |
| 32 | $CH_3$ | CONH— 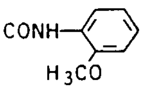 | H | 246-248 |
| 33 | $CH_3$ | CONH— 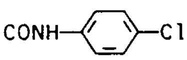 | H | 172-173 |
| 34 | $NHCONHCH_2CH_3$ | CN | H | >250 |
| 35 | $N(COC_6H_5)_2$ | CN | H | 192-194 |
| 36 | $NH-C_6H_5$ | $CONH_2$ | H | >240 |
| 37 | $NH-C_6H_5$ | CN | H | >240 |
| 38 | $NH-CH_2C_6H_5$ | $CONH_2$ | H | 250-252 |
| 39 | $NH-(CH_2)_3CH_3$ | $CONH_2$ | H | 217-219 |
| 40 | 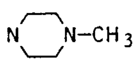 | CN | H | 145-148 |
| 41 | $NHNH-C_6H_5$ | CN | H | 174-176 |
| 42 | $OCH_3$ | CN | H | 202-204 |
| 43 | $S(CH_2)_3CH_3$ | CN | H | 92- 94 |
| 44 | $SCH(CH_3)_2$ | CN | H | 113-114 |
| 45 | $SC(CH_3)_3$ | CN | H | 194-195 |
| 46 | $-NH-N=C(NH_2)-$ | | H | >240 |
| 47 | $NH_2$ | CN | $4,6-(CH_3)_2$ | >300 |
| 48 | 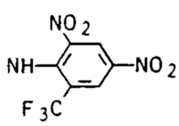 | CN | H | >220 |
| 49 | 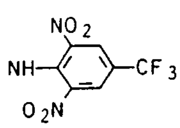 | CN | H | >220 |
| 50 | $(CH_2)_2CH_3$ | $CH_2CH_3$ | H | $Kp_1$ = 130-135 |

a) Literatur:

(1) D.K.J. Gorecki, E.M. Hawes : J. Mech. Chem. 20 (1977) 124

(2) E.M. Hawes, D.G. Wibberley : J. Chem. Soc. C 1967, 1564

(3) K.R. Reddy, K. Mogilaiah, B. Screenivasulu, J. Ind. Chem. Soc. 64 (1987) 193)

Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung,. auf das Wachstum von erwünschten und unerwünschten Pflanzen im Vergleich zum herbiziden Wirkstoff allein wird durch die folgenden biologischen Beispiele aus Gewächshausversuchen belegt.

Als Kulturgefäße dienten Plastikblumentöpfe mit rund 300 $cm^3$ Inhalt und lehmiger Sand mit etwa 3,0 % Humus als Substrat. Die Samen der Testpflanzen wurden nach Arten getrennt flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Sofern die antidotischen Verbindungen als Saatgutbehandlungsmittel eingesetzt wurden, brachte man in den Versuchen eine bestimmte Menge, z.B. 1 g Wirkstoff pro 1 kg Saatgut auf die Samen auf. Diese wurden in die oben beschriebenen Testgefäße eingesät und darin bis zur Nachauflaufbehandlung mit Herbiziden bei einer Wuchshöhe von 3 bis 5 cm angezogen. Die Behandlung mit Herbiziden erfolgte ebenfalls in der oben beschriebenen Weise.

Als Beispielsherbizid der Cyclohexenonderivate der Formel V diente

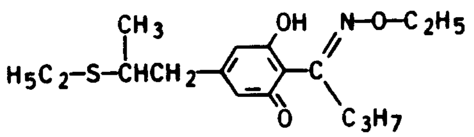

V.2

Der herbizide Wirkstoff V.2 wurde als kommerziell formuliertes Produkt (184 g/l EC auch allein jeweils unter Zugabe von derjenigen Menge an Lösungsmittelsystem XXII - Leerformulierung - ) in die Spritzbrühe gegeben und gemeinsam mit der antidotischen Verbindung ausgebracht.

Sämtliche antidotisch wirkende Verbindungen wurden für die Nachauflaufbehandlung in einem Gemisch, bestehend aus 80 % Cyclohexenon und 20 % Emulphor EL (Versuchsformulierung XXII) mit 10 % (Gewichtsprozent) Wirkstoff aufbereitet.

Bei Saatgutbehandlung wurde die antidotisch wirkende Verbindung als Saatgutpuder mit 40 % (Gewicht) Wirkstoff formuliert und etwas Adhäsionsmittel zugesetzt.

| Liste der Testpflanzen | | |
|---|---|---|
| Lateinischer Name | Deutscher Name | Englischer Name |
| Avena fatua | Flughafer | wild oats |
| Hordeum vulgaris | Gerste | barley |
| Lolium multiflorum | Ital. Raygras | annual raygrass |
| Oryza sativa | Reis | rice |
| Sorghum | Kultursorghum | - |
| Triticum aestivum | Weizen | wheat |
| Zea mays | Mais | Indian corn |

Die Versuchsgefäße wurden im Gewächshaus aufgestellt, wobei für wärmeliebende Arten 18 bis 30 °C und für solche gemäßigter Klimate (10 bis 25 °C) bevorzugt wurden.

Die Versuchsperiode erstreckte sich über 3 bis 5 Wochen. Während dieser Zeit wurden die Pflanzen gepflegt und ihre Reaktion auf die einzelnen Behandlungen wurde erfaßt.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die anschließenden Tabellen dokumentieren die antidotische Wirkung der erfindungsgemäßen Beispielsverbindungen Nr. 1 bei Blatt- und Saatgutbehandlung der Kulturpflanzen sowie der Verbindung Nr. 18, 24 und 44 bei Nachauflauf(Blatt)behandlung. Dabei verbessern diese Verbindungen deutlich die Verträglichkeit des Herbizides V.2 für die Kulturen Weizen, Reis und Mais.

Tabelle 1

| Verbesserung der Verträglichkeit des Herbizids Sethoxydim für Weizen durch Zumischen des Antidots Verbindung Nr. 1 und Nachauflaufanwendung im Gewächshaus | | | |
|---|---|---|---|
| Aufwandmenge [kg/ha] | | Testpflanzen und Schädigung % | |
| Herbizider Wirkstoff V.2 | Antidotische Verbindung 1 | Kulturpflanze Triticum aestivum* | unerwünschte Pflanze Avena fatua |
| 0,03 | - | 45 | 100 |
| 0,06 | - | 94 | 100 |
| 0,03 | 0,125 | 12 | 95 |
| 0,06 | 0,25 | 15 | 100 |

* Sorten: "Kanzler", "Okapi"

Tabelle 2

| Weiteres Beispiel zur Reduktion der Phytotoxizität des Herbizids V.2 gegenüber Weizen durch Zumischen der antidotischen Verbindung Nr. 24 und Nachauflaufanwendung im im Gewächshaus | | | |
|---|---|---|---|
| Aufwandmenge [kg/ha] | | Testpflanzen und Schädigung % | |
| Herbizider Wirkstoff V.2 | Antidotische Verbindung 24 | Kulturpflanze Triticum aestivum* | unerwünschte Pflanze Lolium multifl. |
| 0,015 | - | 30 | 100 |
| 0,03 | - | 65 | 100 |
| 0,06 | - | 90 | 100 |
| 0,015 | 0,06 | 0 | 90 |
| 0,03 | 0,125 | 0 | 100 |
| 0,06 | 0,25 | 35 | 100 |

* Sorten: "Okapi"

Tabelle 3

| Schutz von Kulturpflanzen gegen die Phytotoxizität des Herbizids V.2 durch Saatgutbehandlung mit der antidotischen Verbindung Nr. 1 | | |
|---|---|---|
| Herbizider Wirkstoff V.2 Aufwandmenge [kg/ha] | Antidotische Verbindung 1 Aufwandmenge [g/kg Saatgut] | Schädigung in % bei Zea mays* [Mais] |
| 0,03 | - | 30 |
| 0,06 | - | 65 |
| 0,03 | 1 | 0 |
| 0,06 | 1 | 20 |

* Sorte: "Inrakorn"

Tabelle 4

| Verbesserung der Verträglichkeit des Herbizids V.2 für Weizen durch gemeinsame Anwendung (Gemisch) mit der antidotischen Verbindung Nr. 44 bei Nachauflaufanwendung im Gewächshaus | | | |
|---|---|---|---|
| Aufwandmenge [kg/ha] | | Schädigung [%] | |
| Herbizider Wirkstoff V.2 | Antidotische Verbindung 44 | Triticum aestivum | Lolium multiflorum |
| 0,03 | - | 70 | 98 |
| 0,03 | 0,125 | 25 | 90 |

* Sorte: "Star"

Tabelle 5

| Verbesserung der Verträglichkeit des Herbizids V.2 für Reis durch gemeinsame Anwendung (Gemisch) mit der antidotischen Verbindung Nr. 18 bei Nachauflaufanwendung im Gewächshaus | | | |
|---|---|---|---|
| Aufwandmenge [kg/ha] | | Schädigung [%] | |
| Herbizider Wirkstoff V.2 | Antidotische Verbindung 18 | Oryza sativa* | Echinochloa crus-gallis |
| 0,03 | - | 50 | 90 |
| 0,03 | 0,015 | 10 | 90 |

* Sorte: "Bahia"

**Patentansprüche**

**Patentansprüche für folgende Vertragsstaaten : BE, CH, DE, FR, GB, IT, LI, NL**

**1.** 2, 3-substituierte 1, 8-Naphthyridine der allgemeinen Formel I

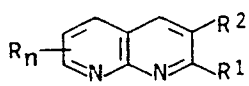

I,

in der die Substituenten die folgende Bedeutung haben:

R — Wasserstoff oder $C_1$-$C_4$-Alkyl (n = 1 oder 2)

$R^1$ — Amino oder $NR^3R^4$, wobei

$R^3$ — $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxy, $C_1$-$C_4$-Halogenalkyl, Nitro, Amino, Mono- oder Dialkylamino mit 1 bis 6 C-Atomen je Alkylrest substituiertes Phenyl oder Phenyl-($C_1$-$C_3$)-alkyl oder eine Gruppe

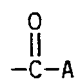

,

worin A für $C_1$-$C_8$-Alkyl, $C_2$-$C_6$-Alkenyl, Phenyl, ein- bis dreifach durch Halogen und/oder $C_1$-$C_4$-Alkyl substituiertes Phenyl, Amino, Alkyl- oder Dialkylamino mit jeweils 1 bis 6 C-Atomen je Alkylrest, Phenylamino, Morpholino oder Piperidyl steht, und

$R^4$ — Wasserstoff, $C_1$-$C_{12}$-Alkyl, $C_3$-$C_8$-Cycloalkyl bedeuten oder

$R^3$ und $R^4$ — miteinander zu einem gesättigten oder ungesättigten 5- oder 6-gliedrigen Heterocyclus, der neben dem Stickstoff, an den sie gebunden sind ein weiteres Heteroatom wie Sauerstoff oder Stickstoff enthalten kann, verbunden sind;

Hydrazino, ein- oder zweifach durch $C_1$-$C_4$-Alkyl oder einfach durch $C_1$-$C_4$-Acyl substituiertes Hydrazino;

$XR^5$, worin X Sauerstoff oder Schwefel bedeutet und $R^5$ für Wasserstoff, $C_1$-$C_{12}$-Alkyl, $C_1$-$C_4$-Alkoxycarbonyl-($C_1$-$C_4$)-alkyl, $C_5$-$C_8$-Cycloakyl, Phenyl, Phenyl-$C_1$-$C_3$-alkyl, Phenyl oder Phenyl-$C_1$-$C_3$-alkyl, jeweils am Ring substituiert durch Halogen, $C_1$-$C_4$-Alkyl, $C_1$-$C_4$-Alkoxy, Amino, Mono- oder Dialkylamino mit 1 bis 6 C-Atomen je Alkylrest steht,

Halogen,

Isothiorhoniumhalogenid

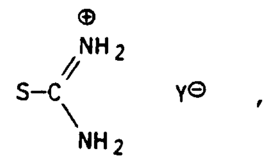

wobei Y für Fluor, Chlor, Brom oder Iod steht,
$C_1$-$C_{12}$-Alkyl,
$C_1$-$C_4$-Alkoxycarbonyl-$C_1$-$C_4$-alkyl, Phenyl oder Phenyl-($C_1$-$C_3$)-alkyl, wobei die aromatischen Reste ein bis drei der folgenden Gruppen tragen können: Halogen, $C_1$-$C_4$-Alkyl, $C_1$-$C_4$-Alkoxy, Amino, Nitro, Mono- oder Dialkylamino mit jeweils 1 bis 4 C-Atomen im Alkylrest,

$R^2$ $C_1$-$C_4$-Alkyl, Cyano,
Carboxyl,
eine Gruppe

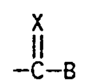 oder 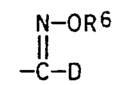 ,

worin

X Sauerstoff oder Schwefel,

B $C_1$-$C_6$-Alkyl, $C_1$-$C_6$-Alkoxy oder Alkylthio, Amino, Mono- oder Dialkylamino mit 1 bis 4 C-Atomen je Alkylrest, Morpholino, Piperidyl, Chlor, Brom oder Phenyl, welches seinerseits ein bis fünf Halogenatome und/oder ein bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy und/oder Alkylthio :

D $C_1$-$C_4$-Alkyl oder $NH_2$ und

$R^6$ Wasserstoff, $C_1$-$C_8$-Alkyl oder $C_2$-$C_8$-Alkylcarbonyl bedeuten oder $R^1$ und $R^2$ gemeinsam $-NH-N=C(NH_2)-$

sowie deren pflanzenverträgliche Salze,
ausgenommen 2-Chlor-1,8-naphthyridin-3-carbonitril, 2-Methoxy-1,8-naphthyridin-3-carbonitril, 2-Methylamino-1,8-naphthyridin-3-carbonitril, 2-Dimethylamino-1,8-naphthyridin-3-carbonitril,2-Diacetylamino-1,8-naphthyridin-3-carbonitril, 2-Phenyl-1,8-naphthyridin-3-carbonitril, 5,7-Dimethyl-2-phenyl-1,8-naphthyridin-3-carbonitril, 2,3,5,7-Tetramethyl-1,8-naphthyridin,2-Methylamino-1,8-naphthyridin-3-carboxamid, 2-Dimethylamino-1,8-naphthyridin-3-carboxamid, 2-Acetyl-amino-1,8-naphthyridin-3-carboxamid, 2-Phenyl-1,8-naphthyridin-3-carboxamid und 2-Amino-1,8-naphthyridin-3-thiocarboxamid,
sowie diejenigen Verbindungen I, bei denen $R^1$ Methyl, Hydroxyl oder Amino ($NH_2$) bedeutet und gleichzeitig $R^2$ für Cyano, Carboxyl, eine Carbonsäureamid- oder Carbonsäureestergruppe steht.

**2.** Verfahren zur Herstellung der Verbindungen I gemäß Anspruch 1, dadurch gekennzeichnet, daß man Aminonicotinaldehyd der Formel II

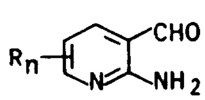 II

mit einer CH-aciden Verbindung der allgemeinen Formel IIIa

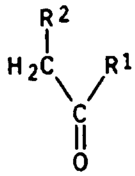

IIIa

oder im Fall von $R^1$ = $NH_2$ oder Hydroxyl mit Malonsäuredinitril oder einem Derivat hiervon (IIIb) in Gegenwart einer Base umsetzt und ggf. anschließend eine Derivatisierung oder Substitution der Reste $R^1$ und/oder $R^2$ in an sich bekannter Weise vornimmt.

**3.** Herbizide Mittel, enthaltend mindestens ein 2,3-substituiertes 1,8-Naphthyridin der allgemeinen Formel I

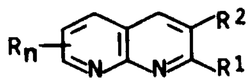

I,

in der die Substituenten die folgende Bedeutung haben:

R Wasserstoff oder $C_1$-$C_4$-Alkyl (n = 1 oder 2) ;

$R^1$ Amino oder $NR^3R^4$, wobei

$R^3$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl oder Phenyl-($C_1$-$C_3$)-alkyl oder eine Gruppe

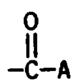

,

worin A für $C_1$-$C_8$-Alkyl, $C_2$-$C_6$-Alkenyl, Phenyl, ein- bis dreifach durch Halogen und/oder $C_1$-$C_4$-Alkyl, substituiertes Phenyl, Amino, Alkyl- oder Dialkylamino mit jeweils 1 bis 6 C-Atomen je Alkylrest, Phenylamino, Morpholino oder Piperidyl steht, und

$R^4$ Wasserstoff, $C_1$-$C_{12}$-Alkyl, $C_3$-$C_8$-Cycloalkyl bedeuten oder

$R^3$ und $R^4$ miteinander zu einem gesättigten oder ungesättigten 5- oder 6-gliedrigen Heterocyclus, der neben dem Stickstoff, an den sie gebunden sind ein weiteres Heteroatom wie Sauerstoff oder Stickstoff enthalten kann, verbunden sind;

Hydrazino, ein- oder zweifach durch $C_1$-$C_4$-Alkyl oder einfach durch $C_1$-$C_4$-Acyl substituiertes Hydrazino;

$XR^5$, worin X Sauerstoff oder Schwefel bedeutet und $R^5$ für Wasserstoff, $C_1$-$C_{12}$-Alkyl, $C_1$-$C_4$-Alkoxycarbonyl-($C_1$-$C_4$)-alkyl, $C_5$-$C_8$-Cycloalkyl, Phenyl, Phenyl-$C_1$-$C_3$-alkyl, Phenyl oder Phenyl-$C_1$-$C_3$-alkyl, jeweils am Ring substituiert durch Halogen, $C_1$-$C_4$-Alkyl, $C_1$-$C_4$-Alkoxy, Amino, Mono- oder Dialkylamino mit 1 bis 6 C-Atomen je Alkylrest steht,

Halogen,

Isothiorhoniumhalogenid,

$C_1$-$C_{12}$-Alkyl,

$C_1$-$C_4$-Alkoxycarbonyl-$C_1$-$C_4$-alkyl, Phenyl oder Phenyl-($C_1$-$C_3$)-alkyl, wobei die aromatischen Reste ein bis drei der folgenden Gruppen tragen können: Halogen, $C_1$-$C_4$-Alkyl, $C_1$-$C_4$-Alkoxy, Amino, Nitro, Mono- oder Dialkylamino mit jeweils 1 bis 4 C-Atomen im Alkylrest;

$R^2$ $C_1$-$C_4$-Alkyl, Cyano,

Carboxyl,

eine Gruppe

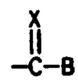 oder 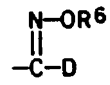 ,

worin

X Sauerstoff oder Schwefel,

B $C_1$-$C_6$-Alkyl, $C_1$-$C_6$-Alkoxy oder Alkylthio, Amino, Mono- oder Dialkylamino mit 1 bis 4 C-Atomen je Alkylrest, Morpholino, Piperidyl, Chlor, Brom oder Phenyl, welches seinerseits ein bis fünf Halogenatome und/oder ein bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy und/oder $C_1$-$C_4$-Alkylthio;

D $C_1$-$C_4$-Alkyl oder $NH_2$ und

$R^6$ Wasserstoff, $C_1$-$C_8$-Alkyl oder $C_2$-$C_8$-Alkylcarbonyl bedeuten oder $R^1$ und $R^2$ gemeinsam $-NH-N=C(NH_2)-$

sowie deren pflanzenverträglichen Salze
und mindestens einen herbiziden Wirkstoff aus der Gruppe

a) der 2-(4-Heteroaryloxy)- oder 2-(4-Aryloxy)-phenoxyessigsäure-derivate der Formel IV,

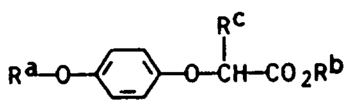 IV,

in der die Substituenten folgende Bedeutung haben:

$R^a$ die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, $C_1$-$C_4$-Alkyl, $C_1$-$C_4$-Halogenalkyl und/oder Nitro;

$R^b$ Wasserstoff, eine $C_1$-$C_4$-Alkylgruppe oder das Äquivalent eines pflanzenverträglichen Kations und

$R^c$ Wasserstoff oder eine Methylgruppe und/oder

b) der Cyclohexenonderivate der Formel V,

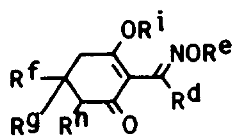 V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann; ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;

einen 10-gliedrigen gesättigten oder einfach ungesättigter Heterocyclus, welcher zwei Sauerstoffatome oder Schwefelatome enthält und durch bis zu drei $C_1$-$C_4$-Alkylgruppen und/oder Methoxygruppen substituiert sein kann;

die Phenylgruppe, die Pyridylgruppe, eine Thiazolylgruppe, eine Pyrazolylgruppe, die Pyrrolylgruppe oder eine Isoxazolylgruppe, wobei diese Gruppen bis zu drei der folgenden Reste tragen können:

$C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen

und/oder Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, die Cyanogruppe, ein Halogenatom oder eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

**4.** Herbizides Mittel nach Anspruch 3, enthaltend ein 2,3-substituiertes 1,8-Naphthyridin I gemäß Anspruch 3 und ein Herbizid a), wobei das Anteilsverhältnis 4:1 bis 0,01:1 Gew.-Teile beträgt.

**5.** Herbizides Mittel nach Anspruch 3, enthaltend ein 2,3-substituiertes 1,8-Naphthyridin I gemäß Anspruch 3 und ein Herbizid b), wobei das Anteilsverhältnis 4:1 bis 0,01:1 Gew.-Teile beträgt.

**6.** Verfahren zur selektiven Bekämpfung von unerwünschtem Pflanzenwuchs, dadurch gekennzeichnet, daß man ein 2,3-substituiertes 1,8-Naphthyridin der Formel I gemäß Anspruch 3 und ein 2-(4-Heteroaryloxy)- oder 2-(4-Aryloxy)-phenoxyessigsäurederivat der Formel IV gemäß Anspruch 3 vor, bei oder nach der Aussaat der Kulturpflanzen, vor oder während des Auflaufens der Kulturpflanzen gleichzeitig oder nacheinander ausbringt.

**7.** Verfahren zur selektiven Bekämpfung von unerwünschtem Pflanzenwuchs, dadurch gekennzeichnet, daß man ein 2,3-substituiertes 1,8-Naphthyridin der Formel I gemäß Anspruch 3 und ein Cyclohexenonderivat der Formel V gemäß Anspruch 3 vor, bei oder nach der Aussaat der Kulturpflanzen, vor oder während des Auflaufens der Kulturpflanzen gleichzeitig oder nacheinander in beliebiger Reihenfolge ausbringt.

**8.** Verfahren zur Verhinderung von Schädigungen von Kulturpflanzen durch herbizide 2-(4-Heteroaryloxy)- oder 2-(4-Aryloxy)-phenoxyessigsäurederivate der Formel IV gemäß Anspruch 3, dadurch gekennzeichnet, daß man das Saatgut der Kulturpflanzen mit einer antagonistisch wirksamen Menge eines 2,3-substituierten 1,8-Naphthyridins der Formel I gemäß Anspruch 3 behandelt.

**9.** Verfahren zur Verhinderung von Schädigungen von Kulturpflanzen durch herbizide Cyclohexenonderivate der Formel V gemäß Anspruch 3, dadurch gekennzeichnet, daß man das Saatgut der Kulturpflanzen mit einer antagonistisch wirksamen Menge eines 2,3-substituierten 1,8-Naphthyridins der Formel I gemäß Anspruch 3 behandelt.

**10.** Verfahren zur selektiven Bekämpfung von unerwünschtem Pflanzenwuchs, dadurch gekennzeichnet, daß man die Blätter und Sprosse der Kulturpflanzen und der unerwünschten Pflanzen im Nachauflaufverfahren mit einem 2,3-substituierten 1,8-Naphthyridin der Formel I gemäß Anspruch 3 und a) mit einem 2-(4-Heteroaryloxy)- oder 2-(4-Aryloxy)-phenoxyessigsäurederivat IV oder b) mit einem Cyclohexenonderivat V, gemäß Anspruch 3 gleichzeitig oder nacheinander behandelt.

**11.** Verfahren nach den Ansprüchen 6 bis 10, dadurch gekennzeichnet, daß die Kulturpflanzen Gerste, Weizen, Mais, Kultursorghum und Reis sind.

**Patentansprüche für folgenden Vertragsstaat : ES**

**1.** Verfahren zur Herstellung von 2,3-substituierten 1,8-Naphthyridinen der allgemeinen Formel I

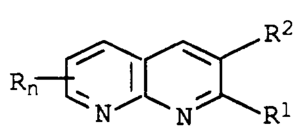

I,

in der die Substituenten die folgende Bedeutung haben:

R Wasserstoff oder $C_1$-$C_4$-Alkyl (n = 1 oder 2);

$R^1$ Amino oder $NR^3R^4$, wobei

$R^3$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis

dreifach durch Halogen, $C_1$-$C_4$-Alkyl, $C_1$-$C_4$-Alkoxy, $C_1$-$C_4$-Halogenalkyl, Nitro, Amino, Mono- oder Dialkylamino mit 1 bis 6 C-Atomen je Alkylrest substituiertes Phenyl oder Phenyl-($C_1$-$C_3$)-alkyl oder eine Gruppe

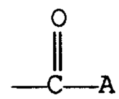 ,

worin A für $C_1$-$C_8$-Alkyl, $C_2$-$C_6$-Alkenyl, Phenyl, ein- bis dreifach durch Halogen und/oder $C_1$-$C_4$-Alkyl substituiertes Phenyl, Amino, Alkyl- oder Dialkylamino mit jeweils 1 bis 6 C-Atomen je Alkylrest, Phenylamino, Morpholino oder Piperidyl steht, und

$R^4$ Wasserstoff, $C_1$-$C_{12}$-Alkyl, $C_3$-$C_8$-Cycloalkyl bedeuten oder

$R^3$ und $R^4$ miteinander zu einem gesättigten oder ungesättigten 5- oder 6-gliedrigen Heterocyclus, der neben dem Stickstoff, an den sie gebunden sind ein weiteres Heteroatom wie Sauerstoff oder Stickstoff enthalten kann, verbunden sind;

Hydrazino, ein- oder zweifach durch $C_1$-$C_4$-Alkyl oder einfach durch $C_1$-$C_4$-Acyl substituiertes Hydrazino;

$XR^5$, worin X Sauerstoff oder Schwefel bedeutet und $R^5$ für Wasserstoff, $C_1$-$C_{12}$-Alkyl, $C_1$-$C_4$-Alkoxycarbonyl-($C_1$-$C_4$)-alkyl, $C_5$-$C_8$-Cycloalkyl, Phenyl, Phenyl-$C_1$-$C_3$-alkyl, Phenyl oder Phenyl-$C_1$-$C_3$-alkyl, jeweils am Ring substituiert durch Halogen, $C_1$-$C_4$-Alkyl, $C_1$-$C_4$-Alkoxy, Amino, Mono- oder Dialkylamino mit 1 bis 6 C-Atomen je Alkylrest steht,

Halogen,

Isothiorhoniumhalogenid

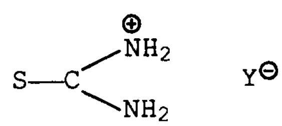 ,

wobei Y für Fluor, Chlor, Brom oder Iod steht,

$C_1$-$C_{12}$-Alkyl,

$C_1$-$C_4$-Alkoxycarbonyl-$C_1$-$C_4$-alkyl, Phenyl oder Phenyl-($C_1$-$C_3$)-alkyl, wobei die aromatischen Reste ein bis drei der folgenden Gruppen tragen können: Halogen, $C_1$-$C_4$-Alkyl, $C_1$-$C_4$-Alkoxy, Amino, Nitro, Mono- oder Dialkylamino mit jeweils 1 bis 4 C-Atomen im Alkylrest,

$R^2$ $C_1$-$C_4$-Alkyl, Cyano, Carboxyl, eine Gruppe

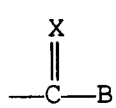 oder 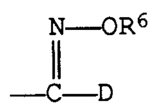 ,

worin

X Sauerstoff oder Schwefel,

B $C_1$-$C_6$-Alkyl, $C_1$-$C_6$-Alkoxy oder Alkylthio, Amino, Mono- oder Dialkylamino mit 1 bis 4 C-Atomen je Alkylrest, Morpholino, Piperidyl, Chlor, Brom oder Phenyl, welches seinerseits ein bis fünf Halogenatome und/oder ein bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy und/oder $C_1$-$C_4$-Alkylthio;

D $C_1$-$C_4$-Alkyl oder $NH_2$ und

$R^6$ Wasserstoff, $C_1$-$C_8$-Alkyl oder $C_2$-$C_8$-Alkylcarbonyl

bedeuten

oder $R^1$ und $R^2$ gemeinsam -NH-N=C ($NH_2$) -

sowie deren pflanzenverträgliche Salze,

ausgenommen 2-Chlor-1,8-naphthyridin-3-carbonitril, 2-Methoxy-1,8-naphthyridin-3-carbonitril, 2-Methylamino-1,8-naphthyridin-3-carbonitril, 2-Dimethylamino-1,8-naphthyridin-3-carbonitril, 2-Diacetylamino-1,8-naphthyridin-3-carbonitril, 2-Phenyl-1,8-naphthyridin-3-carbonitril, 5,7-Dimethyl-2-phenyl-1,8-naphthyridin-3-carbonitril, 2,3,5,7-Tetramethyl-1,8-naphthyridin, 2-Methylamino-1,8-naphthyridin-3-carboxamid, 2-Dimethylamino-1,8-naphthyridin-3-carboxamid, 2-Acetylamino-1,8-naphthyridin-3-carboxamid, 2-Phenyl-1,8-naphthyridin-3-carboxamid und 2-Amino-1,8-naphthyridin-3-thiocarboxamid,

sowie diejenigen Verbindungen I, bei denen $R^1$ Methyl, Hydroxyl oder Amino ($NH_2$) bedeutet und gleichzeitig $R^2$ für Cyano, Carboxyl, eine Carbonsäureamid- oder Carbonsäureestergruppe steht,

dadurch gekennzeichnet, daß man Aminononicotinaldehyd der Formel II

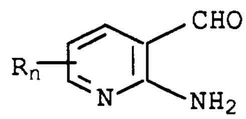

II

mit einer CH-aciden Verbindung der allgemeinen Formel IIIa

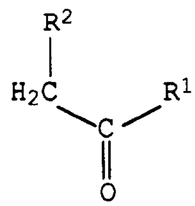

IIIa

oder im Fall von $R^1$ = $NH_2$ oder Hydroxyl mit Malonsäuredinitril oder einem Derivat hiervon (IIIb) in Gegenwart einer Base umsetzt und ggf. anschließend eine Derivatisierung oder Substitution der Reste $R^1$ und/oder $R^2$ in an sich bekannter Weise vornimmt.

**2.** Herbizide Mittel, enthaltend mindestens ein 2,3-substituiertes 1,8-Naphthyridin der allgemeinen Formel I

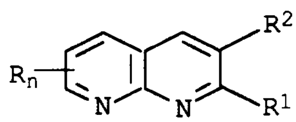

I,

in der die Substituenten die folgende Bedeutung haben:

R Wasserstoff oder $C_1$-$C_4$-Alkyl (n = 1 oder 2);

$R^1$ Amino oder $NR^3R^4$, wobei

$R^3$ $C_1$-$C_{12}$-Alkyl, $C_3$-$C_6$-Alkenyl, $C_3$-$C_8$-Cycloalkyl, Phenyl, Phenyl-($C_1$-$C_3$)-alkyl, ein- bis dreifach durch Halogen, $C_1$-$C_4$-Alkyl, $C_1$-$C_4$-Alkoxy, Amino, Mono- oder Dialkylamino mit 1 bis 6 C-Atomen je Alkylrest substituiertes Phenyl oder Phenyl-($C_1$-$C_3$)-alkyl oder eine Gruppe

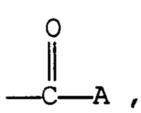

worin A für $C_1$-$C_8$-Alkyl, $C_2$-$C_6$-Alkenyl, Phenyl, ein- bis dreifach durch Halogen und/oder $C_1$-$C_4$-Alkyl, substituiertes Phenyl, Amino, Alkyl- oder Dialkylamino mit jeweils 1 bis 6 C-Atomen je Alkylrest, Phenylamino, Morpholino oder Piperidyl steht, und

| | |
|---|---|
| $R^4$ | Wasserstoff, $C_1$-$C_{12}$-Alkyl, $C_3$-$C_8$-Cycloalkyl bedeuten oder |
| $R^3$ und $R^4$ | miteinander zu einem gesättigten oder ungesättigten 5- oder 6-gliedrigen Heterocyclus, der neben dem Stickstoff, an den sie gebunden sind ein weiteres Heteroatom wie Sauerstoff oder Stickstoff enthalten kann, verbunden sind; |

Hydrazino, ein- oder zweifach durch $C_1$-$C_4$-Alkyl oder einfach durch $C_1$-$C_4$-Acyl substituiertes Hydrazino;

$XR^5$, worin X Sauerstoff oder Schwefel bedeutet und $R^5$ für Wasserstoff, $C_1$-$C_{12}$-Alkyl, $C_1$-$C_4$-Alkoxycarbonyl-($C_1$-$C_4$)-alkyl, $C_5$-$C_8$-Cycloalkyl, Phenyl, Phenyl-$C_1$-$C_3$-alkyl, Phenyl oder Phenyl-$C_1$-$C_3$-alkyl, jeweils am Ring substituiert durch Halogen, $C_1$-$C_4$-Alkyl, $C_1$-$C_4$-Alkoxy, Amino, Mono- oder Dialkylamino mit 1 bis 6 C-Atomen je Alkylrest steht,

Halogen,

Isothiorhoniumhalogenid,

$C_1$-$C_{12}$-Alkyl,

$C_1$-$C_4$-Alkoxycarbonyl-$C_1$-$C_4$-alkyl, Phenyl oder Phenyl-($C_1$-$C_3$)-alkyl, wobei die aromatischen Reste ein bis drei der folgenden Gruppen tragen können: Halogen, $C_1$-$C_4$-Alkyl, $C_1$-$C_4$-Alkoxy, Amino, Nitro, Mono- oder Dialkylamino mit jeweils 1 bis 4 C-Atomen im Alkylrest;

| | |
|---|---|
| $R^2$ | $C_1$-$C_4$-Alkyl, Cyano, Carboxyl, eine Gruppe |

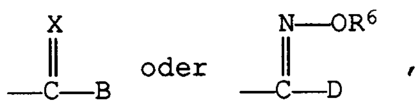

worin

| | |
|---|---|
| X | Sauerstoff oder Schwefel, |
| B | $C_1$-$C_6$-Alkyl, $C_1$-$C_6$-Alkoxy oder Alkylthio, Amino, Mono- oder Dialkylamino mit 1 bis 4 C-Atomen je Alkylrest, Morpholino, Piperidyl, Chlor, Brom oder Phenyl, welches seinerseits ein bis fünf Halogenatome und/oder ein bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy und/oder $C_1$-$C_4$-Alkylthio; |
| D | $C_1$-$C_4$-Alkyl oder $NH_2$ und |
| $R^6$ | Wasserstoff, $C_1$-$C_8$-Alkyl oder $C_2$-$C_8$-Alkylcarbonyl bedeuten |

oder $R^1$ und $R^2$ gemeinsam $-NH-N=C(NH_2)-$
sowie deren pflanzenverträglichen Salze,
und mindestens einen herbiziden Wirkstoff aus der Gruppe

a) der 2-(4-Heteroaryloxy)- oder 2-(4-Aryloxy)-phenoxyessigsäurederivate der Formel IV,

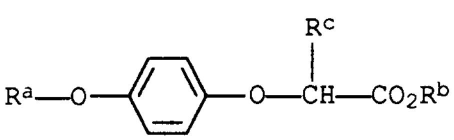

IV,

in der die Substituenten folgende Bedeutung haben:

| | |
|---|---|
| $R^a$ | die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, $C_1$-$C_4$-Alkyl, $C_1$-$C_4$-Halogenalkyl und/oder Nitro; |
| $R^b$ | Wasserstoff, eine $C_1$-$C_4$-Alkylgruppe oder das Äquivalent eines pflanzenverträglichen Kations und |
| $R^c$ | Wasserstoff oder eine Methylgruppe und/oder |

b) der Cyclohexenonderivate der Formel V,

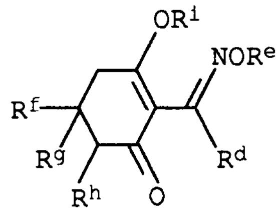

V

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche ein- oder zweifach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;
die Phenylgruppe, die Pyridylgruppe, eine Thiazolylgruppe, eine Pyrazolylgruppe, die Pyrrolylgruppe oder eine Isoxazolylgruppe, wobei diese Gruppen bis zu drei der folgenden Reste tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, die Cyanogruppe, ein Halogenatom oder eine $C_1$-$C_4$-Alkoxycarbonylgruppe oder eine $C_1$-$C_4$-Alkylketoximgruppe und

$R^i$ Wasserstoff oder das Äquivalent eines umweltverträglichen Kations.

**3.** Herbizides Mittel nach Anspruch 2, enthaltend ein 2,3-substituiertes 1,8-Naphthyridin I gemäß Anspruch 2 und ein Herbizid a) oder b), wobei das Anteilsverhältnis 4:1 bis 0,01:1 Gew.-Teile beträgt.

**4.** Verfahren zur selektiven Bekämpfung von unerwünschtem Pflanzenwuchs, dadurch gekennzeichnet, daß man ein 2,3-substituiertes 1,8-Naphthyridin der Formel I gemäß Anspruch 2 und a) ein 2-(4-Heteroaryloxy)- oder 2-(4-Aryloxy)-phenoxyessigsäurederivat der Formel IV oder b) ein Cyclohexenonderivat der Formel V gemäß Anspruch 2, vor, bei oder nach der Aussaat der Kulturpflanzen, vor oder während des Auflaufens der Kulturpflanzen gleichzeitig oder nacheinander ausbringt.

**5.** Verfahren zur Verhinderung von Schädigungen von Kulturpflanzen durch a) herbizide 2-(4-Heteroaryloxy)- oder 2-(4-Aryloxy)-phenoxyessigsäurederivate der Formel IV oder durch b) herbizide Cyclohexenonderivate der Formel V, gemäß Anspruch 2, dadurch gekennzeichnet, daß man das Saatgut der Kulturpflanzen mit einer antagonistisch wirksamen Menge eines 2,3-substituierten 1,8-Naphthyridins der Formel I gemäß Anspruch 2 behandelt.

**6.** Verfahren zur selektiven Bekämpfung von unerwünschtem Pflanzenwuchs, dadurch gekennzeichnet, daß man die Blätter und Sprosse der Kulturpflanzen und der unerwünschten Pflanzen im Nachauflaufverfahren mit einem 2,3-substituierten 1,8-Naphthyridin der Formel I gemäß Anspruch 2 und a) mit einem 2-(4-Heteroaryloxy)- oder 2-(4-Aryloxy)-phenoxyessigsäurederivat IV oder b) mit einem Cyclohexenonderivat V, gemäß Anspruch 2, gleichzeitig oder nacheinander behandelt.

7. Verfahren nach den Ansprüchen 4 bis 6, dadurch gekennzeichnet, daß die Kulturpflanzen Gerste, Weizen, Mais, Kultursorghum und Reis sind.

**Claims**

**Claims for the following Contracting States : BE, CH, DE, FR, GB, IT, LI, NL**

1. A 2,3-substituted 1,8-naphthyridine of the general formula I

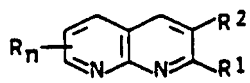

I,

where

R is hydrogen or $C_1$-$C_4$-alkyl (n = 1 or 2),

$R^1$ is amino or $NR^3R^4$, in which

$R^3$ is $C_1$-$C_{12}$-alkyl, $C_3$-$C_6$-alkenyl or $C_3$-$C_8$-cycloalkyl, or phenyl or phenyl-$C_1$-$C_3$-alkyl which is unsubstituted or monosubstituted to trisubstituted by halogen, $C_1$-$C_4$-alkyl, $C_1$-$C_4$-alkoxy, $C_1$-$C_4$-haloalkyl, nitro, amino or mono-or dialkylamino where each alkyl radical is of 1 to 6 carbon atoms, or a group

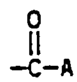

,

where A is $C_1$-$C_8$-alkyl, $C_2$-$C_6$-alkenyl or phenyl which is unsubstituted or monosubstituted to trisubstituted by halogen and/or $C_1$-$C_4$-alkyl, or is amino, alkyl- or dialkylamino where each alkyl radical is of 1 to 6 carbon atoms, phenylamino, morpholino or piperidyl, and

$R^4$ is hydrogen, $C_1$-$C_{12}$-alkyl or $C_3$-$C_8$-cycloalkyl, or

$R^3$ and $R^4$ are bonded to one another to form a saturated or unsaturated 5-membered or 6-membered heterocyclic structure which, in addition to the nitrogen to which they are bonded, may contain a further heteroatom, such as oxygen or nitrogen;

hydrazino which is unsubstituted or monosubstituted or disubstituted by $C_1$-$C_4$-alkyl or monosubstituted by $C_1$-$C_4$-acyl;

$XR^5$, in which X is oxygen or sulfur and $R^5$ is hydrogen, $C_1$-$C_{12}$-alkyl, $C_1$-$C_4$-alkoxycarbonyl-$C_1$-$C_4$-alkyl, $C_5$-$C_8$-cycloalkyl, phenyl, phenyl-$C_1$-$C_3$-alkyl, phenyl or phenyl-$C_1$-$C_3$-alkyl each substituted in the ring by halogen, $C_1$-$C_4$-alkyl, $C_1$-$C_4$-alkoxy, amino or mono- or dialkylamino where each alkyl radical is of 1 to 6 carbon atoms,

halogen,

isothiorhonium halide,

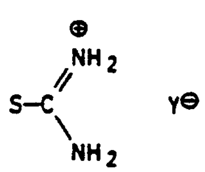

,

where Y is fluorine, chlorine, bromine or iodine,

$C_1$-$C_{12}$-alkyl,

$C_1$-$C_4$-alkoxycarbonyl-$C_1$-$C_4$-alkyl, phenyl or phenyl-$C_1$-$C_3$-alkyl, where the aromatic radicals may carry from one to three of the following groups: halogen, $C_1$-$C_4$-alkyl, $C_1$-$C_4$-alkoxy, amino, nitro or mono- or dialkylamino where each alkyl radical is of 1 to 4 carbon atoms,

$R^2$ is $C_1$-$C_4$-alkyl, cyano, carboxyl or

a group

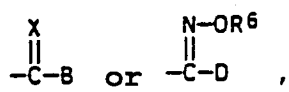

in which

X is oxygen or sulfur,

B is $C_1$-$C_6$-alkyl, $C_1$-$C_6$-alkoxy or alkylthio, amino, mono- or dialkylamino where each alkyl radical is of 1 to 4 carbon atoms, morpholino, piperidyl, chlorine, bromine or phenyl, which in turn may carry from one to five halogen atoms and/or from one to three of the following groups: $C_1$-$C_4$-alkyl, $C_1$-$C_4$-alkoxy, $C_1$-$C_4$-haloalkyl, $C_1$-$C_4$-haloalkoxy and/or $C_1$-$C_4$-alkylthio;

D is $C_1$-$C_4$-alkyl or $NH_2$ and

$R^6$ is hydrogen, $C_1$-$C_8$-alkyl or $C_2$-$C_8$-alkylcarbonyl, or $R^1$ and $R^2$ together are -NH-N=C ($NH_2$) -,

or a plant-tolerated salt thereof,

with the exception of 2-chloro-1,8-naphthyridine-3-carbonitrile, 2-methoxy-1,8-naphthyridine-3-carbonitrile, 2-methylamino-1,8-naphthyridine-3-carbonitrile, 2-di-methylamino-1,8-naphthyridine-3-carbonitrile, 2-diacetylamino-1,8-naphthyridine-3-carbonitrile, 2-phenyl-1,8-naphthyridine-3-carbonitrile, 5,7-dimethyl-2-phenyl-1,8-naphthyridine-3-carbonitrile, 2,3,5,7-tetramethyl-1,8-naphthyridine, 2-methylamino-1,8-naphthyridine-3-carboxamide, 2-dimethylamino-1,8-naphthyridine-3-carboxamide, 2-acetylamino-1,8-naphthyridine-3-carboxamide, 2-phenyl-1,8-naphthyridine-3-carboxamide and 2-amino-1,8-naphthyridine-3-thiocarboxamide,

and those compounds I in which $R^1$ is methyl, hydroxyl or amino ($NH_2$) when $R^2$ is cyano, carboxyl or a carboxamido or carboxylic ester group.

**2.** A process for the preparation of a compound I as claimed in claim 1, wherein an aminonicotinaldehyde of the formula II

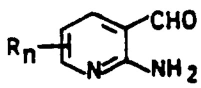

II

is reacted with a CH-acidic compound of the general formula IIIa

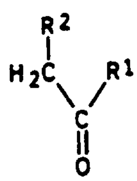

IIIa

or, if $R^1$ is $NH_2$ or hydroxyl, with malonodinitrilo or a derivative thereof IIIb in the presence of a base, and then if appropriate a derivatization or substitution of $R^1$ and/or $R^2$ is carried out in a conventional manner.

**3.** A herbicidal agent containing at least one 2,3-substituted 1,8-naphthyridine of the general formula I

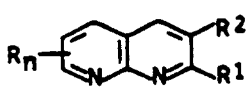

I,

where

R is hydrogen or $C_1$-$C_4$-alkyl (n = 1 or 2),

$R^1$ is amino or $NR^3R^4$, in which

$R^3$ is $C_1$-$C_{12}$-alkyl, $C_3$-$C_6$-alkenyl or $C_3$-$C_8$-cycloalkyl, or phenyl or phenyl-$C_1$-$C_3$-alkyl which is unsubstituted or monosubstituted to trisubstituted by halogen, $C_1$-$C_4$-alkyl, $C_1$-$C_4$-alkoxy, amino or

mono- or dialkylamino where each alkyl radical is of 1 to 6 carbon atoms, or a group

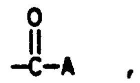

where A is $C_1$-$C_8$-alkyl, $C_2$-$C_6$-alkenyl or phenyl which is unsubstituted or monosubstituted to trisubstituted by halogen and/or $C_1$-$C_4$-alkyl, or is amino, alkyl- or dialkylamino where each alkyl radical is of 1 to 6 carbon atoms, phenylamino, morpholino or piperidyl, and

$R^4$ is hydrogen, $C_1$-$C_{12}$-alkyl or $C_3$-$C_8$-cycloalkyl, or

$R^3$ and $R^4$ are bonded to one another to form a saturated or unsaturated 5-membered or 6-membered heterocyclic structure which, in addition to the nitrogen to which they are bonded, may contain a further heteroatom, such as oxygen or nitrogen;

hydrazino which is unsubstituted or monosubstituted or disubstituted by $C_1$-$C_4$-alkyl or monosubstituted by $C_1$-$C_4$-acyl;

$XR^5$, in which X is oxygen or sulfur and $R^5$ is hydrogen, $C_1$-$C_{12}$-alkyl, $C_1$-$C_4$-alkoxycarbonyl-$C_1$-$C_4$-alkyl, $C_5$-$C_8$-cycloalkyl, phenyl, phenyl-$C_1$-$C_3$-alkyl, phenyl or phenyl-$C_1$-$C_3$-alkyl each substituted in the ring by halogen, $C_1$-$C_4$-alkyl, $C_1$-$C_4$-alkoxy, amino or mono- or dialkylamino where each alkyl radical is of 1 to 6 carbon atoms,

halogen,

isothiorhonium halide,

$C_1$-$C_{12}$-alkyl,

$C_1$-$C_4$-alkoxycarbonyl-$C_1$-$C_4$-alkyl, phenyl or phenyl-$C_1$-$C_3$-alkyl, where the aromatic radicals may carry from one to three of the following groups: halogen, $C_1$-$C_4$-alkyl, $C_1$-$C_4$-alkoxy, amino, nitro or mono- or dialkylamino where each alkyl radical is of 1 to 4 carbon atoms,

$R^2$ is $C_1$-$C_4$-alkyl, cyano, carboxyl or

a group

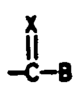 or 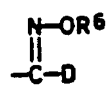 ,

in which

X is oxygen or sulfur,

B is $C_1$-$C_6$-alkyl, $C_1$-$C_6$-alkoxy or alkylthio, amino, mono- or dialkylamino where each alkyl radical is of 1 to 4 carbon atoms, morpholino, piperidyl, chlorine, bromine or phenyl, which in turn may carry from one to five halogen atoms and/or from one to three of the following groups: $C_1$-$C_4$-alkyl, $C_1$-$C_4$-alkoxy, $C_1$-$C_4$-haloalkyl, $C_1$-$C_4$-haloalkoxy and/or $C_1$-$C_4$-alkylthio;

D is $C_1$-$C_4$-alkyl or $NH_2$ and

$R^6$ is hydrogen, $C_1$-$C_8$-alkyl or $C_2$-$C_8$-alkylcarbonyl,

or $R^1$ and $R^2$ together are $-NH-N=C(NH_2)-$,

or a plant-tolerated salt thereof,

and at least one herbicidal active ingredient from the group consisting of

a) 2-(4-hetaryloxy)- or 2-(4-aryloxy)-phenoxyacetic acid derivatives of the formula IV

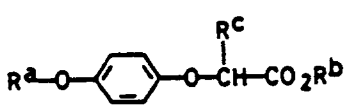

IV,

where

$R^a$ is a phenyl ring, a pyridyl ring, a benzoxazyl radical, a benzothiazyl radical or a benzopyrazinyl radical, where these aromatic ring systems may carry up to two of the following radicals: halogen, $C_1$-$C_4$-alkyl, $C_1$-$C_4$-haloalkyl and/or nitro,

$R^b$ is hydrogen, $C_1$-$C_4$-alkyl or one equivalent of a plant-tolerated cation, and

$R^c$ is hydrogen or methyl, and/or

b) cyclohexenone derivatives of the formula V

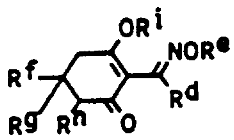

V

where
$R^d$ is $C_1$-$C_4$-alkyl;
$R^e$ is $C_1$-$C_4$-alkyl, $C_3$- or $C_4$-alkenyl, $C_3$- or $C_4$-alkynyl, $C_3$-or $C_4$-haloalkylene or thenyl which may be substituted by a halogen atom;
$R^f$ is $C_1$-$C_4$-alkyl which may be monosubstituted or disubstituted by $C_1$-$C_4$-alkylthio or $C_1$-$C_4$-alkoxy; a 5 -membered or 6-membered saturated or monounsaturated ring system which, in addition to carbon members, may contain an oxygen or sulfur atom or a sulfoxyl or sulfonyl group, and this ring system may carry up to three of the following radicals: hydroxyl, halogen, $C_1$-$C_4$-alkyl, $C_1$-$C_4$-haloalkyl, $C_1$-$C_4$-alkoxy and/or $C_1$-$C_4$-alkylthio;
a 10-membered saturated or monounsaturated heterocyclic structure which contains two oxygen atoms or sulfur atoms and may be substituted by up to three $C_1$-$C_4$-alkyl groups and/or methoxy groups;
phenyl, pyridyl, thiazolyl, pyrazolyl, pyrrolyl or isoxazolyl, where these groups may carry up to three of the following radicals: $C_1$-$C_4$-alkyl, $C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino;
$R^g$ is hydrogen, hydroxyl or, when $R^f$ is $C_1$-$C_6$-alkyl, a $C_1$-$C_6$-alkyl group;
$R^h$ is hydrogen, cyano, halogen, $C_1$-$C_4$-alkoxycarbonyl or $C_1$-$C_4$-alkylketoxime and
$R^i$ is hydrogen or one equivalent of an environmentally compatible cation.

**4.** A herbicidal agent as claimed in claim 3, containing a 2,3-substituted 1,8-naphthyridine I as claimed in claim 3 and a herbicide a), the weight ratio being from 4:1 to 0.01:1.

**5.** A herbicidal agent as claimed in claim 3, containing a 2,3-substituted 1,8-naphthyridine I as claimed in claim 3 and a herbicide b), the weight ratio being from 4:1 to 0.01:1.

**6.** A method for selectively controlling undesirable plant growth, wherein a 2,3-substituted 1,8-naphthyridine of the formula I as claimed in claim 3 and a 2- (4-hetaryloxy)-or 2-(4-aryloxy)-phenoxyacetic acid derivative of the formula IV as claimed in claim 3 are applied, either simultaneously or one after the other, before, during or after sowing of the crops or before or during emergence of the crops.

**7.** A method for selectively controlling undesirable plant growth, wherein a 2,3-substituted 1,8-naphthyridine of the formula I as claimed in claim 3 and a cyclohexenone derivative of the formula V as claimed in claim 3 are applied, either simultaneously or one after the other in any order, before, during or after sowing of the crops or before or during emergence of the crops.

**8.** A method for preventing damage to crops by herbicidal 2- (4-hetaryloxy) - or 2- (4-aryloxy)-phenoxyacetic acid derivatives of the formula IV as claimed in claim 3, wherein the seed of the crops is treated with an antagonistic amount of a 2,3-substituted 1,8-naphthyridine of the formula I as claimed in claim 3.

**9.** A method for preventing damage to crops by herbicidal cyclohexenone derivatives of the formula V as claimed in claim 3, wherein the seed of the crops is treated with an antagonistic amount of a 2,3-substituted 1,8-naphthyridine of the formula I as claimed in claim 3.

**10.** A method for selectively controlling undesirable plant growth, wherein the leaves and the shoots of the crops and the undesirable plants are treated post-emergence with a 2,3-substituted 1,8-naphthyridine of the formula I as claimed in claim 3 and a) with a 2- (4-hetaryloxy)-or 2-(4-aryloxy)-phenoxyacetic acid derivative IV or b) a cyclohexenone derivative V as claimed in claim 3, the compounds being applied

simultaneously or one after the other.

**11.** A method as claimed in any of claims 6 to 10, wherein the crops are barley, wheat, Indian corn, sorghum and rice.

**Claims for the following Contracting State : ES**

**1.** A process for the preparation of a 2,3-substituted 1,8-naphthyridine of the general formula I

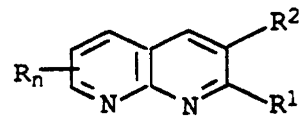

I,

where
R is hydrogen or $C_1$-$C_4$-alkyl (n = 1 or 2),
$R^1$ is amino or $NR^3R^4$, in which
$R^3$ is $C_1$-$C_{12}$-alkyl, $C_3$-$C_6$-alkenyl or $C_3$-$C_8$-cycloalkyl, or phenyl or phenyl-$C_1$-$C_3$-alkyl which is unsubstituted or monosubstituted to trisubstituted by halogen, $C_1$-$C_4$-alkyl, $C_1$-$C_4$-alkoxy, $C_1$-$C_4$-haloalkyl, nitro, amino or mono- or dialkylamino where each alkyl radical is of 1 to 6 carbon atoms, or a group

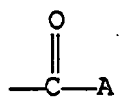

,

where A is $C_1$-$C_8$-alkyl, $C_2$-$C_6$-alkenyl or phenyl which is unsubstituted or monosubstituted to trisubstituted by halogen and/or $C_1$-$C_4$-alkyl, or is amino, alkyl- or dialkylamino where each alkyl radical is of 1 to 6 carbon atoms, phenylamino, morpholino or piperidyl, and
$R^4$ is hydrogen, $C_1$-$C_{12}$-alkyl or $C_3$-$C_8$-cycloalkyl, or
$R^3$ and $R^4$ are bonded to one another to form a saturated or unsaturated 5-membered or 6-membered heterocyclic structure which, in addition to the nitrogen to which they are bonded, may contain a further heteroatom, such as oxygen or nitrogen;
hydrazino which is unsubstituted or monosubstituted or disubstituted by $C_1$-$C_4$-alkyl or monosubstituted by $C_1$-$C_4$-acyl;
$XR^5$, in which X is oxygen or sulfur and $R^5$ is hydrogen, $C_1$-$C_{12}$-alkyl, $C_1$-$C_4$-alkoxycarbonyl-$C_1$-$C_4$-alkyl, $C_5$-$C_8$-cycloalkyl, phenyl, phenyl-$C_1$-$C_3$-alkyl, phenyl or phenyl-$C_1$-$C_3$-alkyl each substituted in the ring by halogen, $C_1$-$C_4$-alkyl, $C_1$-$C_4$-alkoxy, amino or mono- or dialkylamino where each alkyl radical is of 1 to 6 carbon atoms,
halogen,
isothiorhonium halide,

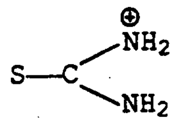

$Y^{\ominus}$ ,

where Y is fluorine, chlorine, bromine or iodine,
$C_1$-$C_{12}$-alkyl,
$C_1$-$C_4$-alkoxycarbonyl-$C_1$-$C_4$-alkyl, phenyl or phenyl-$C_1$-$C_3$-alkyl, where the aromatic radicals may carry from one to three of the following groups: halogen, $C_1$-$C_4$-alkyl, $C_1$-$C_4$-alkoxy, amino, nitro or mono- or dialkylamino where each alkyl radical is of 1 to 4 carbon atoms,
$R^2$ is $C_1$-$C_4$-alkyl, cyano, carboxyl or
a group

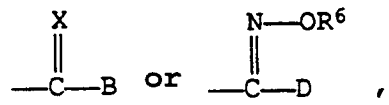

in which

X is oxygen or sulfur,

B is $C_1$-$C_6$-alkyl, $C_1$-$C_6$-alkoxy or alkylthio, amino, mono- or dialkylamino where each alkyl radical is of 1 to 4 carbon atoms, morpholino, piperidyl, chlorine, bromine or phenyl, which in turn may carry from one to five halogen atoms and/or from one to three of the following groups: $C_1$-$C_4$-alkyl, $C_1$-$C_4$-alkoxy, $C_1$-$C_4$-haloalkyl, $C_1$-$C_4$-haloalkoxy and/or $C_1$-$C_4$-alkylthio;

D is $C_1$-$C_4$-alkyl or $NH_2$ and

$R^6$ is hydrogen, $C_1$-$C_8$-alkyl or $C_2$-$C_8$-alkylcarbonyl,

or $R^1$ and $R^2$ together are -NH-N = C($NH_2$)-,

or a plant-tolerated salt thereof,

with the exception of 2-chloro-1,8-naphthyridine-3-carbonitrile, 2-methoxy-1,8-naphthyridine-3-carbonitrile, 2-methylamino-1,8-naphthyridine-3-carbonitrile, 2-dimethylamino-1,8-naphthyridine-3-carbonitrile, 2-diacetylamino-1,8-naphthyridine-3-carbonitrile, 2-phenyl-1,8-naphthyridine-3-carbonitrile, 5,7-dimethyl-2-phenyl-1,8-naphthyridine-3-carbonitrile, 2,3,5,7-tetramethyl-1,8-naphthyridine, 2-methylamino-1,8-naphthyridine-3-carboxamide, 2-dimethylamino-1,8-naphthyridine-3-carboxamide, 2-acetylamino-1,8-naphthyridine-3-carboxamide, 2-phenyl-1,8-naphthyridine-3-carboxamide and 2-amino-1,8-naphthyridine-3-thiocarboxamide,

and those compounds I in which $R^1$ is methyl, hydroxyl or amino ($NH_2$) when $R^2$ is cyano, carboxyl or a carboxamido or carboxylic ester group, wherein an aminonicotinaldehyde of the formula II

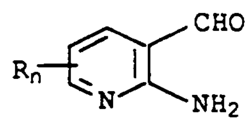

II

is reacted with a CH-acidic compound of the general formula IIIa

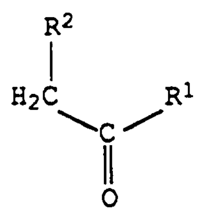

IIIa

or, if $R^1$ is $NH_2$ or hydroxyl, with malonodinitrilo or a derivative thereof (IIIb) in the presence of a base, and then if appropriate a derivatization or substitution of $R^1$ and/or $R^2$ is carried out in a conventional manner.

**2.** A herbicidal agent containing at least one 2,3-substituted 1,8-naphthyridine of the general formula I

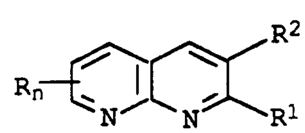

I,

where

R is hydrogen or $C_1$-$C_4$-alkyl (n = 1 or 2),

$R^1$ is amino or $NR^3R^4$, in which

$R^3$ is $C_1$-$C_{12}$-alkyl, $C_3$-$C_6$-alkenyl or $C_3$-$C_8$-cycloalkyl, or phenyl or phenyl-$C_1$-$C_3$-alkyl which is unsubstituted or monosubstituted to trisubstituted by halogen, $C_1$-$C_4$-alkyl, $C_1$-$C_4$-alkoxy, amino or

mono- or dialkylamino where each alkyl radical is of 1 to 6 carbon atoms, or a group

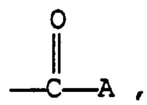

where A is $C_1$-$C_8$-alkyl, $C_2$-$C_6$-alkenyl or phenyl which is unsubstituted or monosubstituted to trisubstituted by halogen and/or $C_1$-$C_4$-alkyl, or is amino, alkyl- or dialkylamino where each alkyl radical is of 1 to 6 carbon atoms, phenylamino, morpholino or piperidyl, and

$R^4$ is hydrogen, $C_1$-$C_{12}$-alkyl or $C_3$-$C_8$-cycloalkyl, or

$R^3$ and $R^4$ are bonded to one another to form a saturated or unsaturated 5-membered or 6-membered heterocyclic structure which, in addition to the nitrogen to which they are bonded, may contain a further heteroatom, such as oxygen or nitrogen;

hydrazino which is unsubstituted or monosubstituted or disubstituted by $C_1$-$C_4$-alkyl or monosubstituted by $C_1$-$C_4$-acyl;

$XR^5$, in which X is oxygen or sulfur and $R^5$ is hydrogen, $C_1$-$C_{12}$-alkyl, $C_1$-$C_4$-alkoxycarbonyl-$C_1$-$C_4$-alkyl, $C_5$-$C_8$-cycloalkyl, phenyl, phenyl-$C_1$-$C_3$-alkyl, phenyl or phenyl-$C_1$-$C_3$-alkyl each substituted in the ring by halogen, $C_1$-$C_4$-alkyl, $C_1$-$C_4$-alkoxy, amino or mono- or dialkylamino where each alkyl radical is of 1 to 6 carbon atoms,

halogen,

isothiorhonium halide,

$C_1$-$C_{12}$-alkyl,

$C_1$-$C_4$-alkoxycarbonyl-$C_1$-$C_4$-alkyl, phenyl or phenyl-$C_1$-$C_3$-alkyl, where the aromatic radicals may carry from one to three of the following groups: halogen, $C_1$-$C_4$-alkyl, $C_1$-$C_4$-alkoxy, amino, nitro or mono- or dialkylamino where each alkyl radical is of 1 to 4 carbon atoms,

$R^2$ is $C_1$-$C_4$-alkyl, cyano, carboxyl or

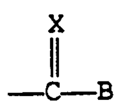 or 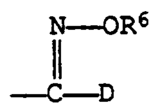 ,

a group in which

X is oxygen or sulfur,

B is $C_1$-$C_6$-alkyl, $C_1$-$C_6$-alkoxy or alkylthio, amino, mono- or dialkylamino where each alkyl radical is of 1 to 4 carbon atoms, morpholino, piperidyl, chlorine, bromine or phenyl, which in turn may carry from one to five halogen atoms and/or from one to three of the following groups: $C_1$-$C_4$-alkyl, $C_1$-$C_4$-alkoxy, $C_1$-$C_4$-haloalkyl, $C_1$-$C_4$-haloalkoxy and/or $C_1$-$C_4$-alkylthio;

D is $C_1$-$C_4$-alkyl or $NH_2$ and

$R^6$ is hydrogen, $C_1$-$C_8$-alkyl or $C_2$-$C_8$-alkylcarbonyl,

or $R^1$ and $R^2$ together are $-NH-N=C(NH_2)-$,

or a plant-tolerated salt thereof,

and at least one herbicidal active ingredient from the group consisting of

a) 2- (4-hetaryloxy) - or 2- (4-aryloxy)-phenoxyacetic acid derivatives of the formula IV

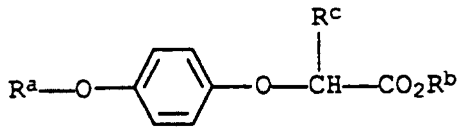 IV,

where

$R^a$ is a phenyl ring, a pyridyl ring, a benzoxazyl radical, a benzothiazyl radical or a benzopyrazinyl radical, where these aromatic ring systems may carry up to two of the following radicals: halogen, $C_1$-$C_4$-alkyl,$C_1$-$C_4$-haloalkyl and/or nitro,

$R^b$ is hydrogen, $C_1$-$C_4$-alkyl or one equivalent of a plant-tolerated cation, and
$R^c$ is hydrogen or methyl, and/or
b) cyclohexenone derivatives of the formula V

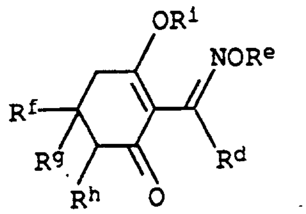

V

where
$R^d$ is $C_1$-$C_4$-alkyl;
$R^e$ is $C_1$-$C_4$-alkyl, $C_3$- or $C_4$-alkenyl, $C_3$- or $C_4$-alkynyl, $C_3$-or $C_4$-haloalkylene or thenyl which may be substituted by a halogen atom;
$R^f$ is $C_1$-$C_4$-alkyl which may be monosubstituted or disubstituted by $C_1$-$C_4$-alkylthio or $C_1$-$C_4$-alkoxy; a 5-membered or 6-membered saturated or monounsaturated ring system which, in addition to carbon members, may contain an oxygen or sulfur atom or a sulfoxyl or sulfonyl group, and this ring system may carry up to three of the following radicals: hydroxyl, halogen, $C_1$-$C_4$-alkyl, $C_1$-$C_4$-haloalkyl, $C_1$-$C_4$-alkoxy and/or $C_1$-$C_4$-alkylthio;
a 10-membered saturated or monounsaturated heterocyclic structure which contains two oxygen atoms or sulfur atoms and may be substituted by up to three $C_1$-$C_4$-alkyl groups and/or methoxy groups;
phenyl, pyridyl, thiazolyl, pyrazolyl, pyrrolyl or isoxazolyl, where these groups may carry up to three of the following radicals: $C_1$-$C_4$-alkyl, $C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino;
$R^g$ is hydrogen, hydroxyl or, when $R^f$ is $C_1$-$C_6$-alkyl, a $C_1$-$C_6$-alkyl group;
$R^h$ is hydrogen, cyano, halogen, $C_1$-$C_4$-alkoxycarbonyl or $C_1$-$C_4$-alkylketoxime and
$R^i$ is hydrogen or one equivalent of an environmentally compatible cation.

**3.** A herbicidal agent as claimed in claim 2, containing a 2,3-substituted 1,8-naphthyridine I as claimed in claim 2 and a herbicide a) or b), the weight ratio being from 4:1 to 0.01:1.

**4.** A method for selectively controlling undesirable plant growth, wherein a 2,3-substituted 1,8-naphthyridine of the formula I as claimed in claim 2 and a) a 2- (4-hetaryloxy)- or 2- (4-aryloxy)-phenoxyacetic acid derivative of the formula IV or b) a cyclohexenone derivative of the formula V as claimed in claim 2 are applied, either simultaneously or one after the other, before, during or after sowing of the crops or before or during emergence of the crops.

**5.** A method for preventing damage to crops by a) herbicidal 2-(4-hetaryloxy)- or 2-(4-aryloxy)-phenoxyacetic acid derivatives of the formula IV or b) herbicidal cyclohexenone derivatives of the formula V, as claimed in claim 2, wherein the seed of the crops is treated with an antagonistic amount of a 2,3-substituted 1,8-naphthyridine of the formula I as claimed in claim 2.

**6.** A method for selectively controlling undesirable plant growth, wherein the leaves and the shoots of the crops and the undesirable plants are treated post-emergence with a 2,3-substituted 1,8-naphthyridine of the formula I as claimed in claim 2 and a) with a 2- (4-hetaryloxy)-or 2-(4-aryloxy)-phenoxyacetic acid derivative IV or b) a cyclohexenone derivative V, as claimed in claim 2, the compounds being applied simultaneously or one after the other.

**7.** A method as claimed in any of claims 4 to 6, wherein the crops are barley, wheat, Indian corn, sorghum and rice.

**Revendications**

**Revendications pour les Etats contractants suivants : BE, CH, DE, FR, GB, IT, LI, NL,**

**1.** 1,8-naphtyridines substituées en 2,3 de la formule générale I

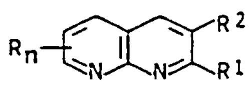 I,

dans laquelle les substituants ont les significations suivantes :

R hydrogène ou alkyle en C1-C4 (n = 1 ou 2)

$R^1$ amino ou $NR^3R^4$,

$R^3$ représentant alkyle en C1-C12, alcényle en C3-C6, cycloalkyle en C3-C8, phényle, phényle-alkyle en C1-C3, phényle substitué une à trois fois par halogène, alkyle en C1-C4, alcoxy en C1-C4, halogénalkyle en C1-C4, nitro, amino, mono- ou dialkylamino avec 1 à 6 atomes-C par reste alkyle, ou phényle-alkyle en C1-C3 ou un groupe

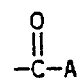 ,

où A est mis pour alkyle en C1-C8, alcényle en C2-C6, phényle, phényle substitué une à trois fois par halogène, et, ou alkyle en C1-C4, amino, alkyle- ou dialkylamino ayant chacun 1 à 6 atomes C par reste alkyle, phénylamino, morpholino ou pipéridyle et

$R^4$ représente hydrogène, alkyle en C1-C12, cycloalkyle en C3-C8 ou

$R^3$ et $R^4$ sont reliés entre eux en un hétérocycle saturé ou non à 5 ou 6 chaînons qui, à côté de l'azote auquel ils sont liés, peut contenir un autre hétéroatome tel qu'oxygène ou azote;

hydrazino, hydrazino substitué une ou deux fois par alkyle en C1-C4 ou une fois par acyle en C1-C4

$XR^5$, où X signifie oxygène ou soufre et $R^5$, hydrogène, alkyle en C1-C12, (alcoxy en C1-C4)-carbonyle-(alkyle en C1-C4), cycloalkyle en C5-C8, phényle, phényle-alkyle en C1-C3, chacun substitué au cycle par halogène, alkyle en C1-C4, alcoxy en C1-C4, amino, mono ou dialkylamino avec 1 à 6 atomes C par reste alkyle,

halogène

halogènure d'isothiorhonium

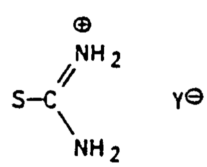 ,

Y étant mis pour fluor, chlore, brome ou iode

alkyle en C1-C12

(alcoxy en C1-C4)-carbonyle-alkyle en C1-C4, phényle ou phénylealkyle en C1-C3, les restes aromatiques pouvant porter un à trois des groupes suivants : halogène, alkyle en C1-C4, alcoxy en C1-C4, amino, nitro, mono ou dialkylamino, ayant chacun 1 à 4 atomes C dans le reste alkyle

$R^2$ alkyle en C1-C4, cyano,

carboxyle

un groupe

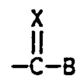 ou 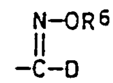 ,

où X représente oxygène ou soufre
B représente alkyle en C1-C6, alcoxy en C1-C6 ou alkylthio, amino, mono- ou dialkylamino avec 1 à 4 atomes-C par reste alkyle, morpholino, piperidyle, chlore, brome ou phényle qui, à son tour, peut porter un à cinq atomes d'halogène et/ou un à trois des groupes suivants : alkyle en C1-C4, alcoxy en C1-C4, halogénalkyle en C1-C4, halogénalcoxy en C1-C4 et/ou alkylthio en C1-C4
D représente alkyle en C1-C4 ou $NH_2$ et
$R^6$, hydrogène, alkyle en C1-C8 ou (alkyle en C2-C8)-carbonyle ou $R^1$ et $R^2$ ensemble -NH-N = C ($NH_2$)-

ainsi que leurs sels acceptables par les plantes
à l'exception de 2-chlore-1,8-naphtyridine-3-carbonitrile, 2-méthoxy-1,8-naphtyridine-3-carbonitrile, 2-méthylamino-1,8-naphtyridine-3-carbonitrile, 2-diméthylamino-1,8-naphtyridine-3-carbonitrile, 2-diacétylamino-1,8-naphtyridine-3-carbonitrile, 2-phényle-1,8-naphtyridine-3-carbonitrile, 5,7-diméthyle-2-phényle-1,8-naphtyridine-3-carbonitrile, 2,3,5,7-tétraméthyle-1,8-naphtyridine, 2-méthylamino-1,8-naphtyridine-3-carboxamide, 2-diméthylamino-1,8-naphtyridine-3-carboxamide, 2-acétyl-amino-1,8-naphtyridine-3-carboxamide, 2-phényle-1,8-naphtyridine-3-carboxamide et 2-amino-1,8-napthyridine-3-thio-carboxamide,
ainsi que ceux des composés I dans lesquels $R^1$ est mis pour méthyle, hydroxyle ou amino ($NH_2$) et simultanément $R^2$ est mis pour cyano, carboxyle, un groupe amide d'acide carboxylique ou ester d'acide carboxylique.

2. Procédé de préparation des composés I selon la revendication 1, caractérisé par le fait que l'on fait réagir, en présence d'une base, de l'aldéhyde d'amino nicotine de la formule II

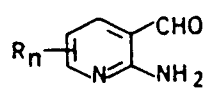 II

avec un composé d'acides en CH de la formule générale IIIa

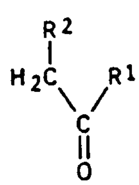 IIIa

ou, dans le cas où $R^1 = NH_2$ ou hydroxyle, avec du dinitrile d'acide malonique ou un dérivé de celui-ci IIIb et, éventuellement ensuite on procède à une dérivation ou substitution des restes $R^1$ et/ou $R^2$ de manière connue en soi.

3. Agent herbicide contenant au moins une 1,8-naphtyridine substituée en 2,3 de la formule générale I

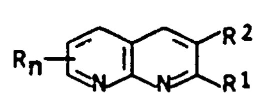 I,

dans laquelle les substituants ont les significations suivantes :
- R hydrogène ou alkyle en C1-C4 (n = 1 ou 2)
- $R^1$ amino ou $NR^3R^4$,

$R^3$ représentant alkyle en C1-C12, alcényle en C3-C6, cycloalkyle en C3-C8, phényle, phényle-alkyle en C1-C3, phényle substitué une à trois fois par halogène, alkyle en C1-C4, alcoxy en C1-C4, halogénalkyle en C1-C4, nitro, amino, mono- ou dialkylamino avec 1 à 6 atomes-C par reste alkyle, ou phényle-alkyle en C1-C3 ou un groupe

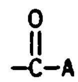 ,

où A est mis pour alkyle en C1-C8, alcényle en C2-C6, phényle, phényle substitué une à trois fois par halogène, et, ou alkyle en C1-C4, amino, alkyle- ou dialkylamino ayant chacun 1 à 6 atomes C par reste alkyle, phénylamino, morpholino ou pipéridyle et

$R^4$ représente hydrogène, alkyle en C1-C12, cycloalkyle en C3-C8 ou

$R^3$ et $R^4$ sont reliés entre eux en un hétérocycle saturé ou non à 5 ou 6 chaînons qui, à côté de l'azote auquel ils sont liés, peut contenir un autre hétéroatome tel qu'oxygène ou azote;

hydrazino, hydrazino substitué une ou deux fois par alkyle en C1-C4 ou une fois par acyle en C1-C4

$XR^5$, où X signifie oxygène ou soufre et $R^5$, hydrogène alkyle en C1-C12, (alcoxy en C1-C4)-carbonyle-(alkyle en C1-C4), cycloalkyle en C5-C8, phényle, phényle-alkyle en C1-C3, chacun substitué au cycle par halogène, alkyle en C1-C4, alcoxy en C1-C4, amino, mono ou dialkylamino avec 1 à 6 atomes C par reste alkyle,

halogène

halogènure d'isothiorhonium

alkyle en C1-C12

(alcoxy en C1 -C4)-carbonyle-alkyle en C1-C4, phényle ou phényle-alkyle en C1-C3, les restes aromatiques pouvant porter un à trois des groupes suivants : halogène, alkyle en C1-C4, alcoxy en C1-C4, amino, nitro, mono ou dialkylamino, ayant chacun 1 à 4 atomes C dans le reste alkyle

$R^2$ alkyle en C1-C4, cyano,

carboxyle

un groupe

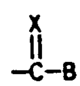 ou 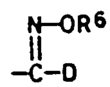

ou X représente oxygène ou soufre

B représente alkyle en C1-C6, alcoxy en C1-C6 ou alkylthio, amino, mono- ou dialkylamino avec 1 à 4 atomes-C par reste alkyle, morpholino, piperidyle, chlore, brome ou phényle qui, à son tour, peut porter un à cinq atomes d'halogène et/ou un à trois des groupes suivants : alkyle en C1-C4, alcoxy en C1-C4, halogénalkyle en C1-C4, halogénalcoxy en C1-C4 et/ou alkylthio en C1-C4

D représente alkyle en C1-C4 ou $NH_2$ et

$R^6$, hydrogène, alkyle en C1-C8 ou (alkyle en C2-C8)-carbonyle ou $R^1$ et $R^2$ ensemble -NH-N = C ($NH_2$)-

ainsi que leurs sels acceptables par les plantes

ainsi qu'au moins un principe actif herbicide des groupes

a) des dérivés d'acide 2-(4-hétéroaryloxy)-ou 2-(4-aryloxy) phénoxyacétique de la formule IV

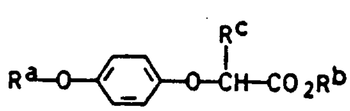 IV,

dans laquelle les substituants ont la signification suivante

$R^a$ un groupe phényle, pyridyle, benzoxazyle, benzothiazyle ou benzopyraginyle, ces systèmes cycliques aromatiques pouvant porter jusqu'à deux des restes suivants : halogène, alkyle en C1-C4, halogénoalkyle en C1-C4 et/ou nitro

$R^b$ hydrogène, un groupe alkyle en C1-C4 ou l'équivalent d'un cation acceptable par les plantes et

$R^c$ hydrogène ou un groupe méthyle et/ou

b) les dèrivés de cyclohexénone de formule V

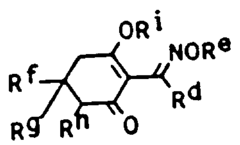

V

dans laquelle, les substituants ont la signification suivante

$R^d$ un groupe alkyle en C1-C4

$R^e$ un groupe alkyle en C1-C4, alcényle en C3-C4, alcinyle en C3-C4, halogénoalkylène en C3-C4, ou un groupe phényle, qui peut être substitué par un atome d'halogène

$R^f$ un groupe alkyle en C1-C4 qui peut être substitué une ou deux fois par alkylthio en C1-C4 ou alcoxy en C1-C4, un système cyclique à 5 ou 6 chaînons, saturé ou non saturé qui peut contenir, à côté de chaînes carbone un atome d'oxygène ou de soufre ou un gourpe sulfoxyde ou sulfone, ce cycle pouvant porter jusqu'à trois des restes suivants hydroxy, halogène, alkyle en C1-C4, halogénalkyle en C1-C4, alcoxy en C1-C4 et/ou alkylthio en C1-C4, un hétérocycle à 10 chaînons, saturé ou non saturé une fois, qui contient deux atomes d'oxygène ou de soufre et qui peut être substitué par jusqu'à trois groupes alkyle en C1-C4 et/ou méthoxy

un groupe phényle, pyridyle, thiazolyle, pyrazolyle, pyrolyle ou isoxazolyle, ces groupes pouvent porter jusqu'à trois des restes suivants:

alkyle en C1-C4, halogénalkyle en C1-C4, alcoxy en C1-C4, alkylthio en C1-C4, alcényloxy en C3-C6, alcinyloxy en C3-C6, alcoxy en C1-C4-alkyle en C1-C3, dialcoxy en C1-C4-alkyle en C1-C3, formyle, halogène et/ou benzoylamino,

$R^g$ hydrogène, hydroxy ou, quand Rf signifie groupe alkyle en C1-C6, un groupe alkyle en C1-C6

$R^h$ hydrogène, un groupe cyano, un atome d'halogène ou un groupe (alcoxy en C1-C4)-carbonyle ou un groupe (alkyle en C1-C4) cétoxime et

$R^i$ hydrogène ou l'équivalent d'un cation acceptable pour l'environnement.

4. Agent herbicide selon la revendication 3 contenant une 1,8-naphtyridine I substituée en 2,3 selon la revendication 3 et un herbicide a) le rapport des parties en poids étant de 4/1 à 0,01/1.

5. Agent herbicide selon la revendication 3 contenant une 1,8-naphtyridine I substituée en 2,3 selon la revendication 3 et un herbicide b), le rapport des parties en poids étant de 4/1 à 0,01/1.

6. Procédé pour lutter sélectivement contre une croissance indésirable des plantes, caractérisé par le fait que l'on projette, avant, pendant ou après l'ensemencement des plantes, avant ou pendant la levée des plantes, simultanément ou successivement une 1,8-naphtyridine substituée en 2,3 de formule I selon la revendication 3 et un dérivé d'acide 2-(4-hétéroaryloxy) ou 2-(4-aryloxy)-phénoxyacétique de formule IV selon la revendication 3.

7. Procédé pour lutter sélectivement contre une croissance indésirable des plantes, caractérisé par le fait que l'on projette, avant, pendant ou après l'ensemencement des plantes, avant ou pendant la levée des plantes, simultanément ou successivement une 1,8-naphtyridine substituée en 2,3 de formule I selon la revendication 3 et un dérivé de cyclohexénone de la formule V selon la revendication 3.

8. Procédé pour empêcher des dommages aux plantes de culture par les dérivés herbicides d'acide 2-(4-hétéroaryloxy)-ou 2-(4-aryloxy)phénoxyacétique de formule IV selon la revendication 3, caractérisé par le fait que l'on traite les semences des plantes de culture avec une quantité à effet antagoniste d'une 1,8-naphtyridine substituée en 2,3 de formule I selon la revendication 3.

**9.** Procédé pour empêcher des dommages aux plantes de culture par les dérivés herbicides de cyclohexénone de formule V selon la revendication 3, caractérisé par le fait que l'on traite les semences des plantes de culture avec une quantité à effet antagoniste d'une 1,8-naphtyridine substituée en 2,3 de formule I selon la revendication 3.

**10.** Procédé de lutte sélective contre la croissance des plantes indésirables, caractérisé par le fait que l'on traite les feuilles et les pousses des plantes de culture et les plantes indésirables en processus de post-émergence, simultanément ou successivement avec une 1,8-naphtyridine substituée sur 2,3 de formule I selon la revendication 3 et a) avec un dérivé d'acide 2-(4-hétéroaryloxy)- ou 2-(4-aryloxy)-phénoxyacétique IV ou b) un dérivé de cyclohexénone V selon la revendication 3.

**11.** Procédé selon les revendications 6 à 10, caractérisé par le fait que les plantes de culture sont l'orge, le blé, le maïs, le sorgho et le riz.

**Revendications pour l'Etat contractant suivant : ES**

**1.** Procédé de préparation de 1,8-naphtyridines substitués en 2,3 de la formule générale I

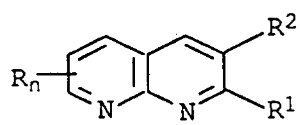

I,

dans laquelle les substituants ont les significations suivantes :

R hydrogène ou alkyle en C1-C4 (n = 1 ou 2)

$R^1$ amino ou $NR^3R^4$,

$R^3$ représentant alkyle en C1-C12, alcényle en C3-C6, cycloalkyle en C3-C8, phényle, phényle-alkyle en C1-C3, phényle substitué une à trois fois par halogène, alkyle en C1-C4, alcoxy en C1-C4, halogénalkyle en C1-C4, nitro, amino, mono- ou dialkylamino avec 1 à 6 atomes-C par reste alkyle, ou phényle-alkyle en C1-C3 ou un groupe

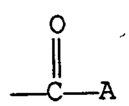

,

où A est mis pour alkyle en C1-C8, alcényle en C2-C6, phényle, phényle substitué une à trois fois par halogène, et, ou alkyle en C1-C4, amino, alkyle- ou dialkylamino ayant chacun 1 à 6 atomes C par reste alkyle, phénylamino, morpholino ou pipéridyle et

$R^4$ représente hydrogène, alkyle en C1-C12, cycloalkyle en C3-C8 ou

$R^3$ et $R^4$ sont reliés entre eux en un hétérocycle saturé ou non à 5 ou 6 chaînons qui, à côté de l'azote auquel ils sont liés, peut contenir un autre hétéroatome tel qu'oxygène ou azote;

hydrazino, hydrazino substitué une ou deux fois par alkyle en C1-C4 ou une fois par acyle en C1-C4

$XR^5$, où X signifie oxygène ou soufre et $R^5$, hydrogène alkyle en C1-C12, (alcoxy en C1-C4)-carbonyle-(alkyle en C1-C4), cycloalkyle en C5-C8, phényle, phényle-alkyle en C1-C3, chacun substitué au cycle par halogène, alkyle en C1-C4, alcoxy en C1-C4, amino, mono ou dialkylamino avec 1 à 6 atomes C par reste alkyle,

halogène

halogènure d'isothiorhonium

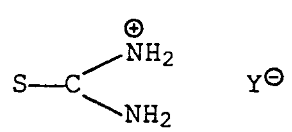

,

Y étant mis pour fluor, chlore, brome ou iode
alkyle en C1-C12
(alcoxy en C1-C4)-carbonyle-alkyle en C1-C4, phényle ou phénylealkyle en C1-C3, les restes aromatiques pouvant porter un à trois des groupes suivants : halogène, alkyle en C1-C4, alcoxy en C1-C4, amino, nitro, mono ou dialkylamino, ayant chacun 1 à 4 atomes C dans le reste alkyle

$R^2$ alkyle en C1-C4, cyano,
carboxyle
un groupe

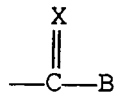 ou 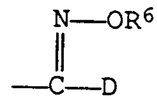 ,

où X représente oxygène ou soufre
B représente alkyle en C1-C6, alcoxy en C1-C6 ou alkylthio, amino, mono- ou dialkylamino avec 1 à 4 atomes-C par reste alkyle, morpholino, piperidyle, chlore, brome ou phényle qui, à son tour, peut porter un à cinq atomes d'halogène et/ou un à trois des groupes suivants : alkyle en C1-C4, alcoxy en C1-C4, halogénalkyle en C1-C4, halogénalcoxy en C1-C4 et/ou alkylthio en C1-C4
D représente alkyle en C1-C4 ou $NH_2$ et
$R^6$, hydrogène, alkyle en C1-C8 ou (alkyle en C2-C8)-carbonyle ou $R^1$ et $R^2$ ensemble -NH-N = C ($NH_2$)-

ainsi que leurs sels acceptables par les plantes
à l'exception de 2-chlore-1,8-naphtyridine-3-carbonitrile, 2-méthoxy-naphtyridine-3-carbonitrile, 2-méthylamino-1,8-naphtyridine-3-carbonitrile, 2-diméthylamino-1,8-naphtyridine-3-carbonitrile, 2-diacétylamino-1,8-naphtyridine-3-carbonitrile, 2-phényle-1,8-naphtyridine-3-carbonitrile, 5,7-diméthyle-2-phényle-1,8-naphtyridine-3-carbonitrile, 2,3,5,7-tétraméthyle-1,8-naphtyridine, 2-méthylamino-1,8-naphtyridine-3-carboxamide, 2-diméthylamino-1,8-naphtyridine-3-carboxamide, 2-acétyl-amino-1,8-naphtyridine-3-carboxamide, 2-phényle-1,8-naphtyridine-3-carboxamide et 2-amino-1,8-napthyridine-3-thio-carboxamide,
ainsi que ceux des composés I dans lesquels $R^1$ est mis pour méthyle, hydroxyle ou amino ($NH_2$) et simultanément $R^2$ est mis pour cyano, carboxyle, un groupe amide d'acide carboxylique ou ester d'acide carboxylique, caractérisé par le fait que l'on fait réagir en présence d'une base de l'aldéhyde d'amino nicotine de la formule II

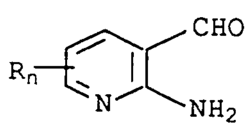

II

avec un composé d'acides en CH de la formule générale IIIa

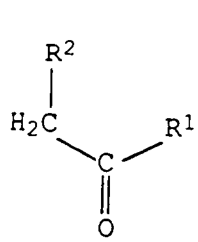

IIIa

ou, dans le cas où $R^1$ = $NH_2$ ou hydroxyle, avec du dinitrile d'acide malonique ou un dérivé de celui-ci IIIb et, éventuellement ensuite on procède à une dérivation ou substitution des restes $R^1$ et/ou $R^2$ de manière connue en soi.

**2.** Agent herbicide contenant au moins une 1,8-naphtyridine substituée en 2,3 de la formule générale I

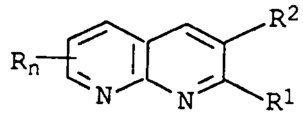

I,

dans laquelle les substituants ont les significations suivantes :

R hydrogène ou alkyle en C1-C4 (n = 1 ou 2)

$R^1$ amino ou $NR^3R^4$,

$R^3$ représentant alkyle en C1-C12, alcényle en C3-C6, cycloalkyle en C3-C8, phényle, phényle-alkyle en C1-C3, phényle substitué une à trois fois par halogène, alkyle en C1-C4, alcoxy en C1-C4, halogénalkyle en C1-C4, nitro, amino, mono- ou dialkylamino avec 1 à 6 atomes-C par reste alkyle, ou phényle-alkyle en C1-C3 ou un groupe

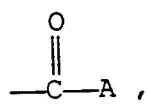

,

ou A est mis pour alkyle en C1-C8, alcényle en C2-C6, phényle, phényle substitué une à trois fois par halogène, et, ou alkyle en C1-C4, amino, alkyle- ou dialkylamino ayant chacun 1 à 6 atomes C par reste alkyle, phénylamino, morpholino ou pipéridyle et

$R^4$ représente hydrogène, alkyle en C1-C12, cycloalkyle en C3-C8 ou

$R^3$ et $R^4$ sont reliés entre eux en un hétérocycle saturé ou non à 5 ou 6 chaînons qui, à côté de l'azote auquel ils sont liés, peut contenir un autre hétéroatome tel qu'oxygène ou azote;

hydrazino, hydrazino substitué une ou deux fois par alkyle en C1-C4 ou une fois par acyle en C1-C4

$XR^5$, où X signifie oxygène ou soufre et $R^5$, hydrogène alkyle en C1-C12, (alcoxy en C1-C4)-carbonyle-(alkyle en C1-C4), cycloalkyle en C5-C8, phényle, phényle-alkyle en C1-C3, chacun substitué au cycle par halogène, alkyle en C1-C4, alcoxy en C1-C4, amino, mono ou dialkylamino avec 1 à 6 atomes C par reste alkyle,

halogène

halogènure d'isothiorhonium

alkyle en C1-C12

(alcoxy en C1-C4)-carbonyle-alkyle en C1-C4, phényle ou phénylealkyle en C1-C3, les restes aromatiques pouvant porter un à trois des groupes suivants : halogène, alkyle en C1-C4, alcoxy en C1-C4, amino, nitro, mono ou dialkylamino, ayant chacun 1 à 4 atomes C dans le reste alkyle

$R^2$ alkyle en C1-C4, cyano,

carboxyle

un groupe

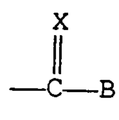

ou

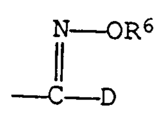

,

où X représente oxygène ou soufre

B représente alkyle en C1-C6, alcoxy en C1-C6 ou alkylthio, amino, mono- ou dialkylamino avec 1 à 4 atomes-C par reste alkyle, morpholino, piperidyle, chlore, brome ou phényle qui, à son tour, peut porter un à cinq atomes d'halogène et/ou un à trois des groupes suivants : alkyle en C1-C4, alcoxy en C1-C4, halogénalkyle en C1-C4, halogénalcoxy en C1-C4 et/ou alkylthio en C1-C4

D représente alkyle en C1-C4 ou $NH_2$ et

$R^6$, hydrogène, alkyle en C1-C8 ou (alkyle en C2-C8)-carbonyle ou $R^1$ et $R^2$ ensemble -NH-N

= C ($NH_2$)-
ainsi que leurs sels acceptables par les plantes
ainsi qu'au moins un principe actif herbicide des groupes

a) des dérivés d'acide 2-(4-hétéroaryloxy)-ou 2-(4-aryloxy) phénoxyacétique de la formule IV

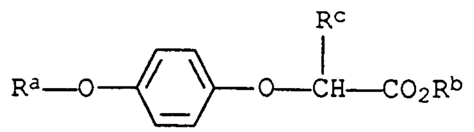

IV,

dans laquelle les substituants ont la signification suivante

$R^a$ un groupe phényle pyridyle, benzoxazyle, benzothiazyle ou benzopyraginyle, ces systèmes cycliques aromatiques pouvant porter jusqu'à deux des restes suivants : halogène, alkyle en C1-C4, halogénoalkyle en C1-C4 et/ou nitro

$R^b$ hydrogène, un groupe alkyle en C1-C4 ou l'équivalent d'un cation acceptable par les plantes et

$R^c$ hydrogène ou un groupe méthyle et/ou

b) les dérivés de cyclohexénone de formule V

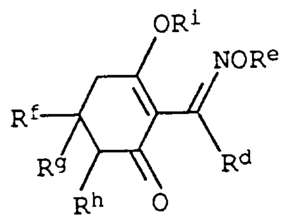

V

dans laquelle, les substituants ont la signification suivante

$R^d$ un groupe alkyle en C1-C4

$R^e$ un groupe alkyle en C1-C4, alcényle en C3-C4, alcinyle en C3-C4, halogénoalkylène en C3-C4, ou un groupe phényle, qui peut être substitué par un atome d'halogène

$R^f$ un groupe alkyle en C1-C4 qui peut être substitué une ou deux fois par alkylthio en C1-C4 ou alcoxy en C1-C4, un système cyclique à 5 ou 6 chaînons, saturé ou non saturé qui peut contenir, à côté de chaînes carbone un atome d'oxygène ou de soufre ou un gourpe sulfoxyde ou sulfone, ce cycle pouvant porter jusqu'à trois des restes suivants hydroxy, halogène, alkyle en C1-C4, halogénalkyle en C1-C4, alcoxy en C1-C4 et/ou alkylthio en C1-C4, un hétérocycle à 10 chaînons, saturé ou non saturé une fois, qui contient deux atomes d'oxygène ou de soufre et qui peut être substitué par jusqu'à trois groupes alkyle en C1-C4 et/ou méthoxy
un groupe phényle, pyridyle, thiazolyle, pyrazolyle, pyrolyle ou isoxazolyle, ces groupes pouvent porter jusqu'à trois des restes suivants :
alkyle en C1-C4, halogénalkyle en C1-C4, alcoxy en C1-C4, alkylthio en C1-C4, alcényloxy en C3-C6, alcinyloxy en C3-C6, alcoxy en C1-C4-alkyle en C1-C3, dialcoxy en C1-C4-alkyle en C1-C3, formyle, halogène et/ou benzoylamino,

$R^g$ hydrogène, hydroxy ou, quand Rf signifie groupe alkyle en C1-C6, un groupe alkyle en C1-C6

$R^h$ hydrogène, un groupe cyano, un atome d'halogène ou un groupe (alcoxy en C1-C4)-carbonyle ou un groupe (alkyle en C1-C4) cétoxime et

$R^i$ hydrogène ou l'équivalent d'un cation acceptable pour l'environnement.

**3.** Agent herbicide selon la revendication 2 contenant une 1,8-naphtyridine I substituée en 2,3 de formule I selon la revendication 2 et un herbicide a) ou b), le rapport des parties en poids étant de 4/1 à 0,01/1.

**4.** Procédé pour lutter sélectivement contre une croissance indésirable des plantes, caractérisé par le fait que l'on projette, avant, pendant ou après l'ensemencement des plantes, avant ou pendant la levée des plantes, simultanément ou successivement une 1,8-naphtyridine substituée en 2,3 de formule I selon la

revendication 2 et a) un dérivé d'acide 2-(4-hètéroaryloxy) ou 2-(4-aryloxy)-phénoxyacétique de formule IV ou b) un dérivé de cyclohexénone de formule V selon la revendication 2.

**5.** Procédé pour protéger les végétaux cultivés contre les dommages provoqués par a) les dérivés herbicides d'acides 2-(4-hétéroaryloxy)- ou 2-(4-aryloxy)-phénoxyacétiques de formule IV ou par b) les dérivés herbicides de cyclohexénone de formule V, selon la revendication 2, caractérisé en ce que l'on traite les semences de la plante cultivée par une quantité antagoniste efficace d'une 1,8-naphtiridine 2,3-substituée de formule I de la revendication 2.

**6.** Procédé pour combattre sélectivement les croissances de végétaux indésirables, caractérisé en ce que l'on traite les feuilles et les pousses des végétaux cultivés et des végétaux indésirables en post-levée, simultanément ou successivement, par une 1,8-naphtiridine 2,3-substituée de formule I de la revendication 2, et a) par un dérivé d'acide 2-(4-hétéroaryloxy)- ou 2-(4-aryloxy)-phénoxyacétique IV ou b) par un dérivé de cyclohexénone V, selon la revendication 2.

**7.** Procédé selon l'une des revendications 4 à 6, caractérisé en ce que les végétaux cultivés consistent en orge, blé, maïs, sorgho cultivé et riz.